# EUROPÄISCHE PATENTSCHRIFT

(11) **EP 2 691 362 B1**
(45) Veröffentlichungstag und Bekanntmachung des Hinweises auf die Patenterteilung: **29.03.2017**
(21) Anmeldenummer: 12705880.8
(22) Anmeldetag: 27.02.2012
(51) Int. Cl.: C07C 67/08, C07C 69/40, C10M 105/36

(54) **GEMISCH VON BERNSTEINSÄUREESTERN ALS WEICHMACHER**
MIXTURE OF SUCCINIC ESTERS AS PLASTICIZER
MÉLANGE D'ESTERS D'ACIDE SUCCINIQUE UTILISÉ COMME PLASTIFIANT

(30) Priorität: 31.03.2011 DE 102011006557
(43) Veröffentlichungstag der Anmeldung: 05.02.2014
(73) Patentinhaber: Evonik Degussa GmbH, 45128 Essen (DE)
(72) Erfinder: BECKER, Hinnerk Gordon, 45257 Essen (DE); GRASS, Michael, 45721 Haltern Am See (DE)
(86) Internationale Anmeldenummer: PCT/EP2012/053285
(87) Internationale Veröffentlichungsnummer: WO 2012/130545

(56) Entgegenhaltungen:
- WO-A1-03/029181
- DE-A1-102006 001 768
- DE-A1-102008 002 168
- Saechtling: "Kunststoff Taschenbuch", 2001, Hanser, XP002678379, ISBN: 3446216057 Seiten 452-455, das ganze Dokument

## Beschreibung

Die vorliegende Erfindung betrifft Gemische von Bernsteinsäureestern, Verfahren zur Herstellung dieser Gemische, Zusammensetzungen enthaltend die Gemische, sowie die Verwendung von Bernsteinsäureestergemischen als solches oder in Zusammensetzungen.

Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung eines Weich-PVC, werden dem PVC Weichmacher zugesetzt, wobei in der überwiegenden Anzahl der Fälle Phthalsäureester, insbesondere Di-2-ethylhexylphthalat (DEHP), Düsononylphthalat (DINP) und Diisodecylphthalat (DIDP) Verwendung finden. Durch bereits bestehende und möglicherweise künftige gesetzliche Regelungen zum Einsatz von Phthalaten besteht der Bedarf, neue als Weichmacher für PVC geeignete Ester zu finden. Darüber hinaus werden PVC-Weichmacher derzeit hauptsächlich aus Rohstoffen aufgebaut, die im Wesentlichen aus der Rohölaufbereitung stammen. Im Hinblick auf die Endlichkeit der Rohölreserven ist eine nachhaltige Nutzung alternativer Quellen geboten. Insbesondere bieten sich diesbezüglich Hydroxyverbindungen (wie z.B. Alkohole) und/oder Carbonsäuren als Weichmacherrohstoffe an. Problem hierbei ist allerdings die schlechte Verfügbarkeit, die schwankende Qualität und die geringe Reinheit der meisten in Frage kommenden "nachwachsenden" Verbindungen, so dass die Auswahl an großtechnisch aktuell oder mittelfristig einsetzbaren "Bio-Rohstoffen" eingeschränkt ist.

Bernsteinsäure ist sowohl petrochemisch (z.B. durch Hydrierung von Maleinsäure) als auch biotechnologisch herstellbar, wobei im letzteren Fall besonders Vorteilhaft nachwachsende Rohstoffe zur Gewinnung der Bernsteinsäure eingesetzt werden können. Bernsteinsäure kommt an zahlreichen Stellen in der freien Natur vor, u.a. als Stoffwechsel-Produkt innerhalb des Citronensäure-Cyclus, in vielen Früchten und Gemüse, Hölzern, Pilzen, Flechten usw.; Bernsteinsäure ist außerdem auch ein Nebenprodukt der alkoholischen Gärung. Eine Reihe von anaeroben Mikroorganismen bildet Bernsteinsäure als Gärungsprodukt aus Zuckern u. Cellulose. Von technischer Bedeutung sind das Pansenbakterium Actinobacillus und das Nicht-Pansenbakterium Anaerobiospirillum, die Bernsteinsäure mit einer Ausbeute von 83-87 % aus Glucose od. Maisquellwasser in Batch-Kulturen herstellen [Römpp Chemielexikon; Onlineausgabe; Zugriff 06/2009].

In der US 6355711 (ExxonMobil; 1998) wird die Herstellung von Weichmachern auf Basis verzweigter Oxo-Alkohole beschrieben. Neben Phthalaten, Adipaten und Trimellitaten wird dabei auch eine Vielzahl anderer Carbonsäureester erwähnt, darunter auch Ester der Bernsteinsäure. Die beschriebenen Alkoholkomponenten weisen allerdings zu mindestens 50 % eine Methylverzweigung am β-Kohlenstoffatom auf, wobei als Hauptkomponenten 3-Methyloctanol, 7-Methyloctanol und 2,6-Dimethylheptanol angegeben werden. Der hohe Anteil an Verzweigungen am β-Kohlenstoffatom, ist allerdings sowohl für die Veresterung als auch die WM-Wirkung nachteilig.

In der DE10043545 (Evonik Oxeno; 2000) wird ein Verfahren zur Herstellung von Carbonsäureestern beschrieben, welche neben anderen Anwendungsgebieten insbesondere auch als Weichmacher für Kunststoffe eingesetzt werden können. Dabei werden u.a. auch Bernsteinsäure als aliphatische Carbonsäure und Isononanol als aliphatischer Alkohol genannt. Die DE10043545 enthält allerdings keine Lehren zur spezifischen Zusammensetzung der Nonanole sowie den spezifischen Materialeigenschaften der Ester bzw. der mit ihnen herstellbaren PVC-Mischungen. Auch bezieht sie sich nicht auf Ester, die auf Basis von nachwachsenden Rohstoffen erzeugt werden.

Gleiches gilt für die in der US2015077 und der US2015088 (DuPont; 1932) beschriebenen Estergemische von polyfunktionellen Carbonsäuren, die auf Basis von aliphatischen verzweigten monofunktionellen Alkoholen hergestellt werden. Als Beispiel für polyfunktionelle Carbonsäuren wird zwar u.a. auch Bernsteinsäure genannt, als Beispiele für monofuktionelle aliphatische verzweigte Alkohole werden u.a. 4,6-Dimethyl-1-heptanol, 4,6-Dimethyl-1-octanol und 3-Methyl-3-octanol genannt. Allerdings sind die genannten Alkohole als Reinstoffe zum einen großtechnisch nicht in ausreichenden Mengen verfügbar, zum Anderen ist beim 3-Methyl-3-octanol, einem sekundären Alkohol, wegen der direkt an der Estergruppe befindlichen Verzweigung ebenfalls von nachteiligen anwendungstechnischen Effekten auszugehen. Weiterhin bezieht sie sich ebenfalls nicht auf Ester, die auf Basis von nachwachsenden Rohstoffen erhältlich sind.

Das Fachbuch Saechting "Kunststoff Taschenbuch", 2001, Hanser (ISBN: 3446216057) beschreibt auf den Seite 452 bis 455 als Weichmacher aus der Gruppe der Ester aliphatischer Dicarbonsäuren unter anderem Di-iso-nonyladipat.

In Bezug auf die Verwendung von nachwachsenden Rohstoffen bei der Herstellung der Weichmacher offenbart die EP 1005562 (Michigan State University; 1998) ein Verfahren zur Herstellung und Aufreinigung von Bernsteinsäure, welche fermentativ aus Kohlehydraten hergestellt wird. Ein direkter Zusammenhang mit bestimmten Bernsteinsäureestern bzw. deren Verwendung als PVC-Weichmacher ergibt sich jedoch nicht.

Die EP 1849764 und die US2006/0252956 (Michigan State University; 2006) beziehen sich auf eine Verfahren zur Herstellung von Carbonsäureestern via reaktiver Destillation, wobei die Säurekomponente bevorzugt auf Basis nachwachsender Rohstoffe ("Biomassen basiert") hergestellt wird. Dabei werden jedoch insbesondere lineare und/oder "niedrige Alkohole" mit 1 bis 8 Kohlenstoffatomen für die Veresterung eingesetzt, im Fokus steht insbesondere Ethanol und insbesondere Ethylester der Zitronensäure. Die Verwendung von Nonylalkoholen wird ebenso wenig beschrieben, wie die Vor- und/oder Nachteile bestimmter Bernsteinsäureester bei der Verwendung als PVC-Weichmacher. Lineare kurzkettige Ester sind flüchtig, längerkettige neigen zur Kristallisation, was die Materialeigenschaften insbesondere in Zusammensetzungen und den daraus hergestellten Produkten negativ beeinflusst.

Die bisher bekannten Bersteinsäureester weisen demnach sowohl technische, ökonomische, toxikologische als auch ökologische Nachteile auf. Technisch ist insbesondere die Flüchtigkeit kurzkettiger aliphatischer Bernsteinsäureester zu hoch, ebenso wie ihre Migrationsgeschwindigkeit. Hinzu kommt bei den längerkettigen Bernsteinsäureestern (insbesondere bei solchen mit linearer Alkoholkomponente) neben einer nicht ausreichenden Gelierung eine schlechte Verträglichkeit mit Polymeren (insbesondere Polyvinylchlorid) und Formulierungsadditiven (wie z.B. Entschäumern, Stabilisatoren, Viskositätsadditiven usw.), die zu einer Trübung im Endprodukt und zu einem Ausschwitzen führt, und einen sinnvollen kommerziellen Einsatz unmöglich macht. Ökonomisch nachteilig ist insbesondere der schwierige Zugang zu Alkoholkomponenten (Reinstoffen) vieler bekannter Bersteinsäureester. Toxikologisch ist insbesondere die Verwendung von Bernsteinsäureestern problematisch, die quartäre Kohlenstoffatome aufweisen, da diese nur schwer biologisch abbaubar sind und daher zur Bioakkumulation neigen. Ökologisch hingegen sind Bernsteinsäureester, die allein auf fossilen petrochemischen Bestandteilen basieren als nicht nachhaltig und wenig zukunftssicher zu bewerten.

Somit sind die bisher im Stand der Technik genannten Bernsteinsäureester aus den vorgenannten Gründen nicht oder nur unzureichend geeignet, um in technisch relevanten Rezepturen einen signifikanten Einsatz zu ermöglichen.

Ausgehend von dem bekannten Stand der Technik bestand daher die Aufgabe, Bernsteinsäureester bereitzustellen, die als Weichmacher für Kunststoffe wie zum Beispiel PVC, PVB oder PAMA Verwendung finden können und bei denen die vorgenannten technischen, ökonomischen, toxikologischen und ökologischen Probleme nicht oder nur in deutlich abgeschwächter Form auftreten.

Die Aufgabe wird gelöst durch ein Gemisch von Bersteinsäureestern, welches sich dadurch auszeichnen, dass der Alkylrest einen Anteil an Alkylkomponenten mit weniger als 9 C-Atomen von maximal 15 Massen-% aufweist, der Alkylrest einen Anteil an Alkylkomponenten mit mehr als 9 C-Atomen von maximal 25 Massen-% aufweist, und wobei der Anteil an 3,5,5-Trimethylhexylresten bei maximal 5 mol-% und der Anteil an linearem n-Nonylresten bei maximal 15 mol-% liegt.

Überraschender Weise wurde gefunden, dass spezielle Gemische isomerer Nonylester der Bernsteinsäure als Weichmacher für Kunststoffe, insbesondere PVC, verwendet werden können, und dort vorteilhafte Eigenschaften gegenüber den bereits literaturbekannten Bernsteinsäure-Estern zeigen, wobei bezüglich der Alkoholkomponente insbesondere ein nur geringer Anteil an höher verzweigten Alkoholen (wie z.B. 3,5,5-Trimethyl-1-hexanol) bei gleichzeitiger Anwesenheit nur beschränkter Anteile an linearem Nonylalkohol, entscheidend für die technische Verwendbarkeit ist.

Bei einer Ausführungsform der Erfindung beträgt der Anteil der Bersteinsäure bezogen auf alle Teile von Bernsteinsäure welche in dem Gemisch als Ester vorliegen, welcher auf nachwachsenden Rohstoffen basiert, mindestens 10 mol-%.

Die Säurekomponente, welche zur Herstellung der erfindungsgemäßen Estergemische verwendet wird ist Bernsteinsäure, wobei der Anteil an Bernsteinsäure, welcher auf nachwachsenden Rohstoffen basiert, im erfindungsgemäßen Herstellverfahren und somit auch im erfindungsgemäßen Estergemisch bis zu 100 mol-% betragen kann.

Bernsteinsäure ist sowohl petrochemisch (z.B. durch Hydrierung von Maleinsäure) als auch biotechnologisch herstellbar, wobei im letzteren Fall besonders Vorteilhaft nachwachsende Rohstoffe zur Gewinnung der Bernsteinsäure eingesetzt werden können.

Bevorzugt beträgt der Anteil an Bernsteinsäure welche unter Verwendung nachwachsender Rohstoffe gewonnen wurde mindestens 10 mol-%, besonders bevorzugt mindestens 30 mol-%, insbesondere bevorzugt mindestens 50 mol-% und insbesondere besonders bevorzugt mindestens 70 mol-% der zur Herstellung der erfindungsgemäßen Estergemische eingesetzten Bernsteinsäure.

Bezüglich der Rohstoffbasis dieser Ausführungsform liegt die Besonderheit der vorliegenden Erfindung in der Nutzung nachwachsender Rohstoffe zur Herstellung der Bernsteinsäure-Gemische. Dabei versteht man im Sinne der vorliegenden Erfindung unter nachwachsenden Rohstoffen im Unterschied zu petrochemischen Rohstoffen, welche auf fossilen Ressourcen, wie z.B. Erdöl oder Steinkohle basieren, solche Rohstoffe, die auf Basis von Biomasse entstehen bzw. hergestellt werden. Die Begriffe "Biomasse", "biobasiert" oder "basierend auf" bzw. "hergestellt aus nachwachsenden Rohstoffen" umfassen alle Materialien biologischen Ursprungs, die dem sogenannten "Kohlenstoff-Kurzzeitzyklus" entstammen, somit nicht Bestandteil geologischer Formationen oder Fossilschichten sind. Insbesondere versteht man unter "auf nachwachsenden Rohstoffen basiert" und "auf Basis nachwachsender Rohstoffe", dass durch die Methode ASTM D6866-08 (¹⁴C-Methode) der entsprechende Anteil an ¹⁴C-Isotopen im Gemisch der Bersteinsäure beziehungsweise im Gemisch der Bernsteinsäureester nachgewiesen werden kann.

Die Identifizierung und Quantifizierung nachwachsender Rohstoffe kann gemäß ASTM-Methode D6866 erfolgen. Kennzeichnend ist u.a. für nachwachsende Rohstoffe ihr Anteil an dem Kohlenstoffisotop ¹⁴C im Gegensatz zu petrochemischen Rohstoffen. Mit Hilfe der Radiokarbonmethode können der Anteil an ¹⁴C-Isotopen und somit auch der Anteil an Molekülen bestimmt werden, welcher auf nachwachsenden Rohstoffen basiert.

Ein Bernsteinsäurenonylester weist in Summe 22 Kohlenstoffatome auf, 2 mal 9 aus den Alkylresten und 4 aus der Bersteinsäure. Bei einem Anteil von biobasierter Bersteinsäure von 100 mol-% ergibt sich somit ein Anteil bezogen auf alle Kohlenstoffatome in Ester von 4/22 = 0,1819. Dieser Faktor variiert mit der entsprechenden Kohlenstoffanzahl der Alkyreste und somit mit dem zur Veresterung eingesetzten Alkoholgemischen. Das Alkoholgemisch kann zur Bestimmung seiner Zusammensetzung mit den gängigen Analyseverfahren (z.B. mittels Gaschromatographie mit anschließender Massenspektroskopie / "GC-MS") entsprechen analysiert werden. Ebenso ist es auch möglich die Anzahl der Kohlenstoffatome in den Alkylresten der Bersteinsäure auch nach der Veresterung (z.B. in dem der Ester zunächst vollständig verseift wird und anschließend die frei werdenden Alkohole analysiert werden) zu bestimmen. Über den maximal möglichen Anteil an biobasierter Bernsteinsäure lässt sich dann der tatsächliche Anteil an biobasierter Bernsteinsäure in Estergemisch errechnen.

Ein besonderer ökonomischer und gleichzeitig ökologischer Vorteil dieser Ausführungsform liegt in der gleichzeitigen Nutzung von nachwachsenden Rohstoffen, als Quelle für die Säurekomponente, und petrochemischen Rohstoffen, als Quelle für das Alkoholgemisch, für die Herstellung der erfindungsgemäßen Bernsteinsäureester, was einerseits eine besonders preiswerte Herstellung und eine breite Anwendbarkeit ermöglicht, andererseits aber auch zu besonders "nachhaltigen" Produkten führt.

In einer weiteren Ausführungsform beträgt der Anteil der Bernsteinsäureester, die mindestens einen 3,5,5-Trimethylhexylrest enthalten, maximal 5 mol-%.

In einer weiteren Ausführungsform enthält das Gemisch einen Anteil an Alkylresten mit 9 Kohlenstoffatomen, die eine Methylverzweigung am zweiten Kohlenstoffatom nach dem Sauerstoff der Carboxylgruppe aufweisen, von maximal 49,5 mol-%.

In einer weiteren Ausführungsform liegt der Kochpunkt des Gemisches höher als 180° C.

In einer weiteren Ausführungsform beträgt die Eigenviskosität des Gemisches bestimmt durch Scherrheometrie mit einer Scherrate von 1/s bei einer Temperatur von 20 °C maximal 40 mPa·s.

Bevorzugt sind die Estergemische hergestellt aus isomeren Nonylalkoholen und Bernsteinsäure wobei das zur Herstellung der Ester verwendete Alkoholgemisch einen Anteil an Alkoholkomponenten mit weniger als 9 Kohlenstoffatomen von maximal 15 Massen-%, und einen Anteil an Alkoholkomponenten mit mehr als 9 Kohlenstoffatomen von maximal 25 Massen-% aufweist, wobei gleichzeitig der Gehalt an 3,5,5-Trimethyl-1-hexanol bei maximal 5 mol-% und der Gehalt an linearem n-Nonanol bei maximal 15 mol % liegt, und die zur Veresterung verwendete Bernsteinsäure auf Basis nachwachsender Rohstoffe hergestellt wurde, oder selbst aus nachwachsenden Rohstoffen gewonnen wurde.

Ein weiterer Gegenstand der vorliegenden Erfindung sind Zusammensetzungen, enthaltend die erfindungsgemäßen Gemische.

In einer Ausführungsform liegt der Anteil an Bernsteinsäureestern auf Basis isomerer Nonylalkohole bei mindestens 60 Massen-%, vorzugsweise bei mindestens 70 Massen-% besonders bevorzugt bei mindestens 80 Massen-%.

In einer anderen Ausführungsform liegt der Anteil an Bernsteinsäureestern auf Basis isomeren Nonylalkohole bei maximal 70 Massen-%, bevorzugt zwischen 1 und 65 Massen-%, besonders bevorzugt zwischen 2 und 55 Massen-% und insbesondere besonders bevorzugt zwischen 5 und 50 Massen-%.

Ebenfalls Gegenstand der vorliegenden Erfindung sind PVC-Zusammensetzungen, insbesondere PVC-Plastisole, dadurch gekennzeichnet, dass sie neben mindestens einem PVC-Homo- oder Copolymere 5 bis 250 Massenanteile des erfindungsgemäßen Estergemisches pro 100 Massenanteile PVC enthalten.

Ebenfalls Gegenstand der vorliegenden Erfindung sind Produkte, Halb- oder Fertigzeuge, Fußbodenbeläge, Wandbeläge, Planen, Folien, Profile, Schläuche, Schutzmassen, Klebstoffe, Dichtstoffe, Isolierungen und Ummantelungen enthaltend die erfindungsgemäßen Bernsteinsäureester.

Die erfindungsgemäßen Bernsteinsäureester weisen eine Säurekomponente, d.h. Bernsteinsäure, auf, die bevorzugt aus nachwachsenden Rohstoffen gewonnen wird. Nachwachsende Rohstoffe im Sinne der vorliegenden Erfindung können dabei alle organische Rohstoffe sein, die beispielsweise aus land- und forstwirtschaftlicher Produktion stammen. Besonders bevorzugt handelt es sich dabei um Kohlenhydrate, insbesondere um Zucker. Die Herstellung der Bernsteinsäure erfolgt dabei bevorzugt über einen Fermentationsprozess wie er beispielsweise aus der EP 1005562 bekannt ist. Dies hat den Vorteil, dass die zur Herstellung verwendeten biochemischen Prozesse besonders energieeffizient arbeiten.

Besonders bevorzugt bestehen die erfindungsgemäßen Bernsteinsäureester aus einer (z.B. biotechnologisch) auf Basis nachwachsender Rohstoffe hergestellten Säurekomponente (Bernsteinsäure) und zwei auf petrochemischer Basis hergestellten Alkoholkomponenten (z.B. isomere Nonylalkohole). Dies hat den Vorteil dass auf diese Weise Bernsteinsäureester zur Verfügung gestellt werden, die in einem ganz besonderen Maße Nachhaltigkeit und Kosteneffizienz bei der Herstellung vereinen.

Vorzugsweise wird die Alkoholkomponente über den Oxo-Prozess, wie er beispielsweise aus der US2327066 bekannt ist gewonnen.

Die Zusammensetzung der zur Herstellung der erfindungsgemäßen Bernsteinsäureester eingesetzten Alkohol-Mischungen lässt sich (z.B. im Rahmen des Oxo-Prozesses) über die Wahl des/der Katalysators/en, die Reaktionsbedingungen, z.B. bei der Oligomerisierung, die verwendeten Rohstoffe, die Prozessführung, z.B. durch Rückführung von Teilströmen, sowie durch eine entsprechende destillative und/oder extraktive Aufarbeitung des Alkohol-Produktstromes variieren und in bestimmten Grenzen gezielt einstellen.

Die zur Herstellung der erfindungsgemäßen Bernsteinsäureester eingesetzten Alkohol-Mischungen weisen insbesondere einen Anteil an Alkoholkomponenten mit weniger als 9 Kohlenstoffatomen von maximal 15 Massen-%, bevorzugt von maximal 14 Massen-%, besonders bevorzugt von maximal 13 Massen-%, und insbesondere bevorzugt von 0 bis 12 Massen-% auf, wobei weitere Untergrenzen zwischen 0 und 7 Massen-%. Zwischen 5 und 9 Massen-% und zwischen 7 und 11 Massen-% liegen. Dies hat den Vorteil, dass der Anteil an höher flüchtigen Komponenten minimiert, und damit die Gesamtflüchtigkeit der erfindungsgemäßen Bernsteinsäureester herabgesetzt wird.

Weiterhin weisen die zur Herstellung der erfindungsgemäßen Bernsteinsäureester eingesetzten Alkohol-Mischungen einen Anteil an Alkoholkomponenten mit mehr als 9 Kohlenstoffatomen von maximal 25 Massen-%, bevorzugt von maximal 23 Massen-%, besonders bevorzugt von maximal 21 Massen-% und insbesondere bevorzugt von 0 bis 20 Massen-% auf, wobei weitere Untergrenzen zwischen 0 und 12 Massen-%, zwischen 6 und 15 Massen-% und zwischen 9 und 20 Massen-% liegen. Dies hat den Vorteil, dass der Anteil an höhermolekularen Komponenten minimiert wird und die Eigenviskosität der erfindungsgemäßen Bernsteinsäureester niedrig ist.

Weiterhin weisen die zur Herstellung der erfindungsgemäßen Bernsteinsäureester eingesetzten Alkohol-Mischungen insbesondere einen Anteil an linearem n-Nonanol von maximal 15 mol-%, bevorzugt vom maximal 14 mol-%, besonders bevorzugt von maximal 13 mol-%, und insbesondere bevorzugt von 0 bis 13 mol-% auf, wobei weitere Untergruppen zwischen 2 und 12 mol-%, zwischen 3 und 11 mol-%, zwischen 2 und 5 mol-% sowie zwischen 4 und 10 mol-% liegen. Dies hat den Vorteil, dass der Anteil an linearen Bernsteinsäureestern, die eine sehr begrenzte Verträglichkeit mit PVC und Formulierungsadditiven sowie eine höhere Flüchtigkeit aufweisen innerhalb der erfindungsgemäßen Bernsteinsäureester minimiert wird.

Weiterhin weisen die zur Herstellung der erfindungsgemäßen Bernsteinsäureester eingesetzten Alkohol-Mischungen insbesondere einen Anteil an isomeren Alkoholen mit 9 Kohlenstoffatomen, die eine Methylverzweigung am β-Kohlenstoffatom des Alkohols aufweisen von maximal 49,5 mol-% auf, bevorzugt von maximal 48 mol-%, besonders bevorzugt von maximal 47 mol %, und insbesondere bevorzugt von 0 bis 45 mol-%, wobei weitere Untergruppen zwischen 0 und 18 mol-%, zwischen 10 und 44 mol-%, zwischen 12 und 42 mol-%, zwischen 14 und 41 mol-%, zwischen 15 und 35 mol-% sowie zwischen 32 und 42 mol-% liegen. Dies hat den Vorteil, dass der Anteil an Alkoholen, die auf Grund von sterischer Hinderung nur langsam verestern gering ist.

Weiterhin weisen die zur Herstellung der erfindungsgemäßen Bernsteinsäureester eingesetzten Alkohol-Mischungen insbesondere einen Anteil an isomeren Alkoholen mit 9 Kohlenstoffatomen, die zwei Methylverzweigungen aufweisen von maximal 49,5 mol-% auf, bevorzugt maximal 48 mol-%, besonders bevorzugt maximal 47 mol-% und insbesondere bevorzugt zwischen 0 und 45 mol-%, wobei weitere Untergruppen zwischen 5 und 44mol-%, zwischen 12 und 25 mol-%, zwischen 14 und 34 mol-% sowie zwischen 36 und 44 mol-% liegen. Dies hat den Vorteil, dass der Anteil an Alkoholen die zu Bernsteinsäureestern mit hoher Eigenviskosität führen minimiert wird.

Weiterhin enthalten die zur Herstellung der erfindungsgemäßen Bernsteinsäureester eingesetzten Gemische isomerer Nonylalkohole der Summenformel C₈H₁₇CH₂OH insbesondere weniger als 10 Mol-%, vorzugsweise weniger als 5 Mol-%, bevorzugt weniger als 1 Mol-% und insbesondere von 0 bis 0,5 Mol-%, bevorzugt weniger als 0,1 Mol-%, insbesondere von 0,0001 bis 0,1 Mol-% und besonders bevorzugt weniger als 0,05 Mol-%, insbesondere von 0,01 bis 0,05 Mol % an 3,5,5-Trimethylhexanol oder anderen dreifach substituierten Nonylalkoholen mit der Summenformel C₈H₁₇CH₂OH, insbesondere solchen mit quartären C-Atomen. Dies hat den Vorteil, dass der Anteil an Alkoholen die zu Bernsteinsäureestern mit hoher Eigenviskosität führen minimiert wird.

Die Isomerenverteilungen der isomeren Nonylalkohole in den Gemischen können mit den üblichen, dem Fachmann geläufigen Messmethoden wie NMR-Spektroskopie, GC- oder GC/MS-Spektroskopie, bevorzugt nach Überführung in die Silyl- oder Methylester, gegebenenfalls nach vorheriger Aufreinigung bzw. Trennung mittels flüssigchromatographischer Methoden (z.B. HPLC) ermittelt werden.

Sehr gute Fließfähigkeit und niedrige Eigenviskosität lassen sich dann erzielen, wenn insbesondere der Anteil an mehrfach verzweigten Molekülen, insbesondere an Trimethyl-1-hexanol möglichst gering gehalten wird. Die erfindungsgemäßen Bernsteinsäureester enthalten daher nur sehr geringe Mengen an Estern, die als Alkoholkomponenten Trimethyl-1-hexanol, wie z.B. 3,5,5-Trimethylhexanol, oder andere Alkoholkomponenten mit quartären C-Atomen enthalten. Insbesondere liegt im erfindungsgemäßen Estergemisch der Anteil an Bernsteinsäureester, der mindestens einen 3,5,5-Trimethylhexylrest enthält, bei maximal 10 Massen-%, bevorzugt bei maximal 8 Massen-%, besonders bevorzugt bei maximal 6 Massen-% und insbesondere bevorzugt bei maximal 5 Massen-%.

Die erfindungsgemäßen Bernsteinsäureester bestehen aus einer Säurekomponente (Bernsteinsäure) und einer Alkoholkomponente (Mischung isomerer Nonylalkohole). Um eine Bernsteinsäureester mit einer möglichst geringen Eigenviskosität zu erhalten, weist das zur Herstellung der Bernsteinsäureester eingesetzte Gemisch isomerer Nonylalkohole insbesondere eine maximale Scherviskosität (bei 20 °C) von 20 mPa*s, bevorzugt von maximal 15 mPa*s und besonders bevorzugt von maximal 12 mPa*s auf.

Die erfindungsgemäßen Gemische von Diisononylsuccinaten bzw. die Diisononylsuccinate selbst können alle dem Stand der Technik bekannten Weisen hergestellt werden, vorzugsweise mit dem nachfolgend beschriebenen Verfahren.

Neben dem Gemisch selbst wird auch ein Verfahren zu dessen Herstellung beansprucht.

Ein solches Verfahren zur Herstellung von zuvor beschriebenen Gemischen umfassend das in Kontakt bringen von Bernsteinsäure oder Bernsteinsäuredimethylester mit einem Gemisch isomerer C9-Alkohole, wobei hierdurch Wasser oder Methanol freigesetzt wird; wobei ein bis zu 50% stöchiometrischer Überschuss des Gemisches isomerer C9-Alkohole Verwendung findet, und die Umsetzung unter Verwendung eines Katalysators erfolgt, insbesondere ausgewählt aus der Gruppe umfassend Butyltitanat, Nonyltitanat.

In einer Variante des Verfahrens beträgt der Anteil der Bersteinsäure oder Bersteinsäurederivaten, welche zur Herstellung des Esters eingesetzt wird und auf nachwachsenden Rohstoffen basiert, mindestens 10 mol-%.

In einer Variante des Verfahrens wird Bernsteinsäure mit einem Gemisch isomerer Nonanole, im Folgenden Isononanol genannt, optional in Gegenwart eines Katalysators unter Freisetzung von Wasser verestert.

In einer weiteren Variante des Verfahrens wird Bernsteinsäuredimethylester, mit einem Gemisch isomerer Nonanole unter Freisetzung von Methanol, optional unter Verwendung eines Katalysators, zum Gemisch isomerer Nonylester der Bernsteinsäure umgeestert.

Eine besondere Ausführungsform des erfindungsgemäßen Verfahrens ist dadurch gekennzeichnet, dass der Anteil an Bernsteinsäurediestern des n-Nonanols im erhaltenen Estergemisch bei maximal 20 Massen-% und der Anteil an Bernsteinsäurediestern des 3,5,5-Trimethylhexanols im erhaltenen Estergemisch bei maximal 10 Massen-% liegt.

Das erfindungsgemäße Verfahren zur Herstellung isomerer Nonylester der Bernsteinsäure zeichnet sich in einer Verfahrensvariante insbesondere dadurch aus, dass Bernsteinsäure oder ein Dialkylbernsteinsäureester, insbesondere Bernsteinsäuredimethylester, mit einem Gemisch isomerer Nonanole umgesetzt wird, wobei ein Katalysator zum Einsatz kommt. Besonders bevorzugt wird bei der Umsetzung von Bernsteinsäure bzw. Bernsteinsäurederivaten mit einem 50% stöchiometrischen Überschuss an Isonoylalkohol(en) gearbeitet. Als Katalysatoren können z.B. Brönstedt und/oder Lewis-Säure eingesetzt werden, insbesondere bevorzugt ist der Einsatz von Schwefellsäure, Methylsulfonsäure, Titanaten und Oxalaten. In einer besonderen bevorzugten Ausführungsform werden entweder Butyltitanat oder Nonyltitanat als Veresterungskatalysator eingesetzt, wobei der Einsatz von Nonyltitanat insbesondere bevorzugt ist, und den Vorteil aufweist, dass sich weniger Nebenprodukte durch Umesterung bilden können. Die Reaktionstemperatur während der Veresterung liegt zwischen 150 und 250 °C, wobei während der Veresterung eine kontinuierliche Abtrennung niedermolekularer Reaktionsprodukte, wie z.B. Wasser, erfolgt. Die Durchführung der Veresterung erfolgt entweder kontinuierlich oder diskontinuierlich, z.B. in Batch-Fahrweise, besonders bevorzugt erfolgt die Veresterung diskontinuierlich. Als Reaktionsbehälter können (nach entsprechender Adaptierung) prinzipiell alle dem Stand der Technik bekannten Reaktortypen verwendet werden, besonders bevorzugt, insbesondere bei diskontinuierlicher Reaktionsführung ist die Verwendung eines Rührkessels. Für den Fall der kontinuierlichen Reaktionsführung kommen bevorzugt Rührkesselkaskaden und/oder Rohrreaktoren zum Einsatz, wobei beim Einsatz letzterer die niedermolekularen Reaktionsprodukte in einem oder mehreren separaten Verfahrensschritten entfernt werden müssen (z.B. durch Kombination des Strömungsrohres mit einem Rührkessel mit Destillationskolonne bzw. durch den Einsatz von Verdampfern. In einer besonderen bevorzugten Ausführungsform wird zum quantitativen Übertreiben der niedermolekularen Komponente(n) ein Hilfsstoff zudosiert, insbesondere bevorzugt ist die Zudosierung von Stickstoffgas. Der Reaktionsfortschritt wird durch die Bestimmung der Säurezahl (gemäß DIN EN ISO 2114) sowie über Gaschromatographie verfolgt, wobei eine Säurezahl von < 0,1 mg KOH/g als Maß für das Erreichen eines vollständigen Umsatzes an Bernsteinsäure gilt. Die Reaktionsdauer beträgt (ab dem Zeitpunkt des Siedebeginnes der Reaktionsmischung) insbesondere zwischen 60 und 500 Minuten, bevorzugt zwischen 70 und 400 Minuten, besonders bevorzugt zwischen 80 und 300 Minuten, und insbesondere bevorzugt zwischen 90 und 250 Minuten. Im Anschluss an die Veresterung erfolgt die Aufarbeitung des Reaktionsgemisches, die die Zersetzung und/oder Abtrennung des verwendeten Veresterungskatalysators sowie die (z.B. destillative) Aufreinigung des Reaktionsgemisches umfasst.

Weiterhin kann auch das Bernsteinsäuredichlorid, welches durch Umsetzung der Bernsteinsäure mit Chlorierungsmitteln, wie beispielweise Thionylchlorid, erhältlich ist, als Ausgangsstoff zur Herstellung der Diisononylester eingesetzt werden.

In einer besonderen bevorzugten Ausführungsform werden als Ausgangssubstanzen zur Herstellung der erfindungsgemäßen Bernsteinsäureester, Bernsteinsäure und/oder Bernsteinsäuremethylester und ein Gemisch isomerer Nonylalkohole - welche aus einem Oxo-Prozeß stammen - eingesetzt. Insbesondere bevorzugt sind dabei solche isomere Nonylalkohole, die durch einen Oxo-Prozess auf Basis von Butenen hergestellt werden, z.B. auf Basis von sog. Crack C₄-Raffinerieschnitten.

Vorzugsweise wird ein Gemisch isomerer Nonanole eingesetzt, welches mindestens zwei Nonanole der Summenformel C₈H₁₇CH₂OH mit unterschiedlicher Konstitutionsformel aufweist, wobei keine der im Gemisch vorhandenen Nonylalkohole einen Anteil von mehr als 50 Mol-%, bevorzugt zumindest 49,5 Mol-%, aufweist.

Die erfindungsgemäßen Gemische weisen eine geringe Flüchtigkeit auf, die in der Endanwendung und/oder während des Herstellprozesses Weichmacherverluste über Verdampfung minimiert. Der Kochpunkt einer Isomerenmischung wird auch durch ihre Zusammensetzung bestimmt und ist in sofern ein charakteristisches Merkmal. Der Kochpunkt unter Atmosphärendruck der erfindungsgemäßen Bernsteinsäureester, bestimmt mittels Differential Scanning Calorimetrie (Tangentenmethode), liegt dabei insbesondere über 180 °C, bevorzugt über 190 °C, besonders bevorzugt über 200 °C und insbesondere bevorzugt über 210 °C.

Die erfindungsgemäßen Gemische weisen eine geringe Eigenviskosität auf, was besonders Vorteilhaft für die Herstellung von Weich-PVC-Pasten ist, da dies auch zu einer geringen Pastenviskosität und damit zu einer besonders guten Fließfähigkeit der Pasten und besonders guten Verarbeitungseigenschaften führt. Die Eigenviskosität der erfindungsgemäßen Bernsteinsäureester liegt deutlich unterhalb der mit der gleichen Alkoholkomponente herstellbaren Phthalate. Insbesondere liegt die Eigenviskosität der erfindungsgemäßen Bernsteinsäureester bestimmt durch Scherrheometrie mit einer Scherrate von 1/s bei einer Temperatur von 20 °C bei maximal 40 mPa*s, bevorzugt bei maximal 35 mPa*s, besonders bevorzugt bei maximal 30 mPa*s und insbesondere bevorzugt bei maximal 25 mPa*. Insbesondere liegt die durch Scherrheometrie (20 °C; Scherrate: 1/s) bestimmte Eigenviskosität zwischen 5 und 30 mPa*s, besonders bevorzugt zwischen 7 und 28 mPa*s und insbesondere bevorzugt zwischen 9 und 25 mPa*s. Dies ist insofern besonders vorteilhaft, als ein deutlicher Viskositätsunterschied beispielsweise zum derzeitigen Standardweichmacher Diisononylphthalat (Eigenviskosität > 70 mPa*s) besteht, der bei der Verwendung in Formulierungen (z.B. in PVC-Pasten) zu deutliche reduzierten Pastenviskositäten führt, und somit schnellere Maschinengeschwindigkeiten (z.B. bei der Streichverarbeitung) ermöglicht.

Die erfindungsgemäßen Gemische sind besonders bevorzugt farblos und transparent, und eignen sich somit besonders (aber nicht ausschließlich) für die Verwendung in (pigmentierten oder unpigmentierten) weißen und/oder transparenten PVC-Anwendungen (z.B. Deckstrich, Kalanderfolien usw.). Insbesondere weisen die erfindungsgemäßen Bernsteinsäureester eine nach Hazen/APHA-Farbzahlskala photometrisch bestimmte Farbzahl von maximal 50, bevorzugt von maximal 45, besonders bevorzugt von maximal 40 und insbesondere bevorzugt von maximal 30 auf. In einer besonderen Ausführungsform liegt die nach der Hazen/APHA-Farbzahlskala photometrisch bestimmte Farbzahl zwischen 1 und 30, bevorzugt zwischen 2 und 25, besonders bevorzugt zwischen 3 und 20 und insbesondere bevorzugt zwischen 4 und 15. Dies hat den Vorteil, dass die erfindungsgemäßen Bernsteinsäureester mit sehr niedrigen Hazen/APHA-Farbzahlen (d.h. < 20) sich insbesondere für den Einsatz in optisch hochwertigen Applikationen eignen. Die niedrigen Farbzahlen werden dabei insbesondere durch den erfindungsgemäßen Herstellprozess sowie die dazu gehörigen (destillativen) Aufarbeitungsschritte realisiert.

Die nach dem erfindungsgemäßen Verfahren (inkl. destillativer Aufarbeitung und Katalysatorabtrennung) hergestellten erfindungsgemäßen Bernsteinsäureester weisen besonders bevorzugt einen niedrigen Restsäuregehalt auf, um Umesterungen, Esterspaltungen und/oder Nebenreaktionen (z.B. mit Stabilisatoren, Additiven usw.) insbesondere in komplexen PVC-Formulierungen zu verhindern. Insbesondere weisen die erfindungsgemäßen Bernsteinsäureester eine nach DIN EN ISO 2114 bestimmte Säurezahl von maximal 1 mg KOH/g, bevorzugt von maximal 0,5 mg KOH/g, besonders bevorzugt von maximal 0,25 mg KOH/g und insbesondere bevorzugt von maximal 0,1 mg KOH/g auf.

Die nach dem erfindungsgemäßen Verfahren (inkl. destillativer Aufarbeitung) hergestellten erfindungsgemäßen Bernsteinsäureester weisen besonders bevorzugt einen niedrigen Restfeuchtegehalt (Wassergehalt) auf, um Zersetzungs- und/oder Nebenreaktionen zu verhindern. Der nach DIN 51777 bestimmte Wassergehalt liegt für die nach dem erfindungsgemäßen Verfahren hergestellten erfindungsgemäßen Bernsteinsäureester bei maximal 1 %, bevorzugt bei maximal 0,5 %, besonders bevorzugt bei maximal 0,1 % und insbesondere bevorzugt beim maximal 0,075 %.

Die Reinheit der erfindungsgemäßen Bernsteinsäureester lässt sich nach dem Stand der Technik mittels Gaschromatographie bestimmen. Dabei muss auf Grund der vorliegenden Isomerenverteilung über die Peakfläche des entsprechenden Peakbereichs integriert werden. Die Reinheit nach dem erfindungsgemäßen Verfahren (inkl. destillativer Aufarbeitung) hergestellten erfindungsgemäßen Bernsteinsäureester liegt in der Regel bei > 98 %, bevorzugt bei > 98,5 %, besonders bevorzugt bei > 99 % und insbesondere bevorzugt bei > 99,25 %. Dadurch wird erreicht, dass ausschließlich klar identifizierte Stoffe für die Weiterverarbeitung (z.B. in PVC-Pasten) eingesetzt werden, was insbesondere bei der Beurteilung der Toxikologie sowie von Verträglichkeitsphänomenen (z.B. Ausschwitzerscheinungen) von Vorteil ist.

Durch die spezielle Struktur der erfindungsgemäßen Bernsteinsäureester werden Moleküle zur Verfügung gestellt, die eine eher niedrige Dichte aufweisen. Dies ist von besonderem Vorteil, da einerseits bei der Verwendung z.B. in PVC-Formulierungen der Weichmacher in Massenanteilen zudosiert wird, andererseits aber Volumenphänomene wie z.B. die Fließeigenschaften insbesondere für die Pastenverarbeitung von hoher Relevanz sind. Je niedriger also die Dichte eines Weichmachers ist, desto stärker trägt er (bei gleichzeitig niedriger Eigenviskosität) zu einer niedrigeren Pastenviskosität bei. Die erfindungsgemäßen Bernsteinsäureester weisen eine nach DIN 51757 bestimmte Dichte bei 20 °C von maximal 0,96 g/cm³ auf, bevorzugt von maximal 0,95 g/cm³, besonders bevorzugt von maximal 0,94 g/cm³ und insbesondere bevorzugt von maximal 0,93 g/cm³ auf.

Gegenüber den aus dem Stand der Technik bekannten Bernsteinsäureestern auf Basis linearer Alkohole weisen die erfindungsgemäßen Isononylester eine weit geringere Flüchtigkeit auf. Auch gegenüber den aus dem Stand der Technik bekannten Bernsteinsäureestern auf Basis verzweigter Alkohole weisen die erfindungsgemäßen Isononylester eine bessere Verarbeitbarkeit und (in vielen Fällen) auch eine geringere Flüchtigkeit auf. Gegenüber den aus dem Stand der Technik bekannten Bernsteinsäureestern auf Basis 2-Ethylhexanol weisen die erfindungsgemäßen Isononylester z.B eine deutlich geringere Flüchtigkeit aus Deckstrichfolie, sowie in Plastisolen einen geringeren Anstieg der Viskosität (Eindickfaktor) mit der Zeit und somit eine verbesserte Alterungsbeständigkeit auf. Die im Vergleich zur den auf Basis 2-Ethylhexanol hergestellten Bernsteinsäureestern verzögerte Gelierung sorgt für einen längere Verarbeitbarkeit bei erhöhten Temperaturen und kann durch Zumischen geringer Anteile eines Schnellgelierers, wie z.B. Alkylbenzoate, Pyrolidonderivate, Citrate usw., an die vorliegenden technischen Bedürfnisse angepasst werden. Die Weichmachende Wirkung, bestimmt durch die nach 24h bestimmte Shore "A"-Härte, liegt dabei nur wenig niedriger als beim Di(2-ethylhexyl)succinat, Gelbwert und Opazität sind vergleichbar.

Gegenüber den aus dem Stand der Technik bekannten Bernsteinsäureestern auf Basis 3,5,5-Trimethyl-1-hexanol weisen die erfindungsgemäßen Isononylester eine deutlich niedrigere Eigenviskosität auf. Auch in Plastisolen ist die Viskosität der erfindungsgemäßen Diisononylester niedriger, so dass sich die erfindungsgemäßen Ester hervorragend zur Herstellung niedrigviskoser Plastisole, insbesondere auch in Abmischung mit anderen Estern eignen. Darüber hinaus wird gegenüber den aus dem Stand der Technik bekannten Di-3,5,5-trimethylhexylsuccinaten eine verbesserte Gelierung und weichmachende Wirkung in oder bei der Herstellung von Kunststoffen bzw. Kunststoffzusammensetzungen erzielt.

Überraschenderweise zeigen die erfindungsgemäßen Gemische in für den Anwender zentralen Eigenschaften auch gegenüber dem entsprechenden Phthalat verbesserte Eigenschaften wie zum Beispiel eine deutlich niedrigere Eigen- und Pastenviskosität, einen niedrigeren Eindickfaktor (d.h. Erhöhung der Pastenviskosität bei Lagerung der Paste z.B. für 2h, 24h und 7 Tage) insbesondere bei hohen Schergeschwindigkeiten, eine deutlich verbesserte Thermostabilität gemessen am Anstieg des Gelbwertes YI sowie der maximalen Verweilzeit bei der Verarbeitungstemperatur (mindestens 160 °C) bis zur Schwarzfärbung, wobei die weichmachende Wirkung bestimmt durch die nach 24h bestimmte Shore "A"- Härte sowie die Opazität und der Gelbwert in Deckstrichrezepturen bei gleichen Weichmacherkonzentrationen gleich ist.

Neben den Gemischen werden auch Zusammensetzungen beansprucht, welche diese Gemische umfassen.
In einer Ausführungsform weist die Zusammensetzung ein Polymer, ausgewählt aus Polyvinylchlorid, Polyvinylbutyral und/oder Polyalkylmethacrylat auf.

In einer Ausführungsform der Erfindung liegt das Verhältnis (Massenanteile) von dem erfindungsgemäßen Gemisch zu dem Polymer in einem Bereich von 1 zu 25 bis 25 zu 1.

In einer weiteren Ausführungsform der Erfindung liegt das Verhältnis des erfindungsgemäßen Bernsteinsäureestergemisches zu anderen, nicht erfindungsgemäßen Weichmachern, in einem Bereich von 1 zu 10 bis 10 zu 1.

Die erfindungsgemäßen Gemische werden entweder alleine, oder in Mischungen mit anderen Weichmachern, beispielsweise Schnellgelierern, als Zusammensetzungen, wie z.B. in Weich-PVC-Pasten, eingesetzt. Erfolgt ein Einsatz zusammen mit anderen Weichmachern, so liegt der Anteil der erfindungsgemäßen Bernsteinsäureester an der Weichmachermischung bei mindestens 5 Massen-%, bevorzugt bei mindestens 15 Massen-%, besonders bevorzugt bei mindestens 25 Massen-% und insbesondere bevorzugt bei mindestens 30 Massen-%. Dies hat den Vorteil, dass durch den gezielten Einsatz der erfindungsgemäßen Bernsteinsäureester insbesondere die Pastenviskosität einer Weich-PVC-Paste gezielt herabgesetzt werden kann.

In einem besonderen Ausführungsfall liegt der Anteil der erfindungsgemäßen Gemische an dem eingesetzten Weichmachergemisch bei mindestens 50 Massen-%, besonders bevorzugt bei mindestens 60 Massen-% und insbesondere bevorzugt bei mindestens 70 Massen-%. Dies hat den Vorteil, dass durch den gezielten Einsatz der erfindungsgemäßen Bernsteinsäureester insbesondere PVC-Pasten mit niedriger Pastenviskosität hergestellt werden können, deren Verarbeitungseigenschaften, beispielsweise der Gelierung, durch Zugabe weiterer Weichmacher, wie z. B. Schnellgelierern, in einer weiten Bandbreite eingestellt werden können.

Die von den Diisononylestern der Bernsteinsäure abweichenden Verbindungen (Weichmacher) können sowohl nieder- wie auch hochmolekular sein und insbesondere sowohl monomere wie auch polymere Materialparameter aufweisen. Für Gemische, die weitere Ester enthalten, sind diese ausgewählt aus Citronensäuretrialkylestern, acylierten Citronensäuretrialkylestern, Glycerinestern, epoxidierten Pflanzenölen, gesättigten oder ungesättigten Fettsäureestern, welche auch partiell oder vollständig epoxidiert sein können, Glykoldibenzoaten, Alkylbenzoaten, Dialkyladipaten, Trialkyltrimellitaten, Dialkylterephthalaten, Dialkylphthalaten oder den Dialkylestern der 1,2-, 1,3- oder 1,4-Cyclohexandicarbonsäuren, wobei die Alkylreste von 4 bis 13, vorzugsweise 5, 6, 7, 8, 9, 10, 11 oder 13 Kohlenstoffatome aufweisen. Die Weichmacher können auch Dianhydrohexitolester, bevorzugt Isosorbiddiester von Carbonsäuren, wie zum Beispiel n- oder iso-Buttersäure, Valeriansäure oder 2-Ethylhexansäure oder Isononansäure sein.

Polymere, welche in der erfindungsgemäßen Zusammensetzung enthalten sein können, sind z. B. Polyvinylchlorid (PVC), Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), CelluloseAcetat/Butyrat (CAB), Polyvinylbutyral (PVB) und die Polyalkylmethacrylate (PAMA) sowie Mischungen oder Copolymere der genannten Polymere. Besonders bevorzugt ist das Polymer Polyvinylchlorid (PVC).

Erfindungsgemäße Zusammensetzungen werden insbesondere dadurch gekennzeichnet, dass das Verhältnis von erfindungsgemäßen Bersteinsäureestern zu Polymer zwischen 1:25 und 25:1 Gewichtsanteilen liegt, bevorzugt zwischen 1:20 und 20:1, besonders bevorzugt zwischen 1:25 und 10:1 und insbesondere bevorzugt zwischen 1:22 und 5:1.

Der Gehalt an erfindungsgemäßen Bernsteinsäureestern in den Zusammensetzungen orientiert sich an der jeweiligen Zielanwendung. So beträgt er für Weich-PVC-Pasten insbesondere zwischen 5 und 70 Gewichtsanteile pro 100 Gewichtsanteile an PVC, bevorzugt zwischen 7 und 65 Gewichtsanteile, besonders bevorzugt zwischen 9 und 60 Gewichtsanteile und insbesondere bevorzugt zwischen 10 und 58 Gewichtsanteile, wobei weitere Untergruppen zwischen 10 und 25 Gewichtsanteilen, zwischen 20 und 35 Gewichtsanteilen sowie zwischen 30 und 55 Gewichtsanteilen liegen.

Für Trockenmischungen, den Dryblends, beträgt der Anteil der erfindungsgemäßen Bernsteinsäureester an den Zusammensetzungen zwischen 10 und 65 Gewichtsanteilen pro 100 Gewichtsanteile an PVC, bevorzugt zwischen 12 und 62 Gewichtsanteilen, besonders bevorzugt zwischen 15 und 60 Gewichtsanteilen und insbesondere bevorzugt zwischen 17 und 58 Gewichtsanteilen, wobei weitere Untergruppen zwischen 18 und 30 Gewichtsanteilen, zwischen 25 und 42 Gewichtsanteilen sowie zwischen 32 und 56 Gewichtsanteilen liegen.

In bevorzugten Gemischen, die Diisononylester der Bernsteinsäure und gleichzeitig strukturell davon abweichende Weichmacher enthalten, beträgt das Massen-Verhältnis von Weichmachern, insbesondere von Alkylbenzoaten, Dialkyladipaten, Citronensäuretrialkylestern, acylierten Citronensäuretrialkylestern, Trialkyltrimellitaten, Glycoldibenzoaten, Glycerinestern, Dialkylterephthalaten, Dialkylphthalaten, Dialkanoylestern des Isosorbid und/oder den Dialkylestern der 1,2-, 1,3- oder 1,4-Cyclohexandicarbonsäuren, zu Diisononylsuccinaten 1 zu 10 bis 10 zu 1, bevorzugt 1 zu 5 bis 5 zu 1.

Die erfindungsgemäßen Formulierungen bzw. Mischungen, welche die erfindungsgemäßen Bernsteinsäureester enthalten, können neben Polymer(en) und/oder weiteren Weichmachern bzw. Estern auch noch weitere Bestandteile enthalten. Diese weiteren Bestandteile sind insbesondere ausgewählt aus Pigmenten, Füllstoffen, Lösungsmitteln, Stabilisatoren, Costabilisatoren (z.B. epoxidierte Pflanzenöle), Rheologieadditiven, Mattierungsmitteln, Treibmitteln, Zersetzungskatalysatoren, Entlüftungsadditiven, Fungiziden und Flammschutzmitteln.

Ebenfalls Gegenstand der vorliegenden Erfindung sind Produkte, welche die erfindungsgemäßen Bernsteinsäureester enthalten, insbesondere Fußbodenbeläge auf Basis PVC, Wandbeläge (z.B. Tapeten) auf Basis von PVC sowie Schläuche, Folien, Halbzeuge, Fertigzeuge und Planen auf Basis von PVC. Diese Produkte weisen auf Grund der Verwendung der erfindungsgemäßen Bernsteinsäureester insbesondere besonders günstige Verarbeitungseigenschaften bzw. auf Grund des Anteils an nachwachsenden Rohstoffen in den verwendeten erfindungsgemäßen Bernsteinsäureestern insbesondere verbesserte Umweltkennzahlen (z.B. CO₂-Bilanz) auf.

In einer besonderen Ausführungsform handelt es sich bei den erfindungsgemäßen Folien um Verpackungsfolien, insbesondere um solche, die für die Verpackung von Lebensmitteln eingesetzt werden, wobei insbesondere die guten toxikologischen Eigenschaften der erfindungsgemäßen Bernsteinsäureester vorteilhaft sind.

Neben den Gemischen werden auch Verwendungen derselben beansprucht.

In einer weiteren besonderen Ausführungsform werden die Folien, welche das erfindungsgemäße Gemische enthalten zur Herstellung von Aufbewahrungsbeuteln für Körperflüssigkeiten (insbesondere Blut oder Urin) verwendet, wobei insbesondere die guten toxikologischen Eigenschaften der erfindungsgemäßen Gemische vorteilhaft sind.

In einer weiteren besonderen Ausführungsform handelt es sich bei den Halbzeugen bzw. Fertigzeugen um Bestandteile von Kinderspielzeug, die sich insbesondere durch ihre guten toxikologischen Eigenschaften auszeichnen. Insbesondere bevorzugt ist bei der Verwendung der erfindungsgemäßen Gemische in Zusammensetzungen, die zur Herstellung von Kinderspielzeug verwendet werden, die Kombination der erfindungsgemäßen Gemische mit Stabilisatoren, die keine Schwermetalle enthalten.

In einer weiteren besonderen Ausführungsform handelt es sich bei den Halbzeugen bzw. Fertigzeugen um Produkte, die für die medizinische Betreuung von Menschen und bzw. oder Tieren verwendet werden (z.B. Beatmungsmaske, Tubus, Katheteranschluss usw.), wobei insbesondere die gute Sterilisierbarkeit der Produkte sowie die guten toxikologischen Eigenschaften der erfindungsgemäßen Gemische vorteilhaft sind.

Es wird auch die Verwendung der erfindungsgemäßen Zusammensetzung beansprucht.

Vorzugsweise wird die erfindungsgemäße Zusammensetzung als Weichmacher verwendet.

In einer Ausführungsform wird die Zusammensetzung als Weichmacher bei der Herstellung von Farben, Tinten, Klebstoffen oder Klebstoffkomponenten, Lacken, Plastisolen, Schutzmassen und/oder Dichtungsmassen verwendet.

In einer Ausführungsform wird die Zusammensetzung als Lösemittel bei der Herstellung von Farben, Tinten, Klebstoffen oder Klebstoffkomponenten, Lacken, Plastisolen, und/oder Dichtungsmassen verwendet.

In einer Ausführungsform wird die Zusammensetzung als Schmierölkomponente verwendet.

In einer Ausführungsform wird die Zusammensetzung als Hilfsmittel bei der Metallverarbeitung verwendet.

Ein weiterer Gegenstand der vorliegenden Erfindung ist die Verwendung der erfindungsgemäßen Gemischen sowie von Zusammensetzungen, die die erfindungsgemäßen Gemische enthalten.

Die Verwendung der Gemische oder der Zusammensatzung kann hierbei in oder als Farben, Tinte(n) oder Lacke(n), in Plastisole(n), Klebstoffe(n) oder Klebstoffkomponente(n), in Dichtungsmassen, in oder als Weichmacher(n) für Kunststoffe oder Kunststoffkomponenten, als Lösemittel, als Schmierölkomponente und als Hilfsmittel bei der Metallverarbeitung erfolgen.

### Analytik:

### 1. Bestimmung des Gehaltes an 3,5,5-Trimethylhexanol und n-Nonanol via Isomerenbestimmung mittels Gaschromatographischer Analyse (GC)

Die Bestimmung des Gehaltes an 3,5,5-Trimethylhexanol und n-Nonanol der zur Herstellung der erfindungsgemäßen Ester verwendeten Gemisches an verzweigten Nonylalkoholen (= Isononanol) mittels Gaschromatographie (GC) erfolgte mit einem GC-Automaten "HP 5896" von Hewlet Packard unter Verwendung einer DB-FFAP-Säule (Länge: 30m, Innendurchmesser: 0,25 mm, Filmdicke 0,25 µm) von Agilent und einem Flammenionisationsdetektor bei folgenden Rahmenbedingungen:

| | |
|---|---|
| GC-Ofentemperatur: 145 °C | Injektortemperatur: 250 °C |
| Detektortemperatur: 250 °C | |
| Gesamtlaufzeit: 50 Minuten | |

| | |
|---|---|
| Trägergas: Helium (1 bar) | Splitfluss: 100 ml/min |
| Injektionsvolumen: 0,2 µl | |

Die Zuordnung der GC-Signale zu den beiden Isomeren erfolgte anhand von Laufzeitvergleichen mit entsprechenden Vergleichssubstanzen.

### 2. Bestimmung der Ester- Reinheit mittels Gaschromatographischer Analyse (GC)

Die Bestimmung der Reinheit der hergestellten Ester mittels GC erfolgt mit einem GC-Automaten "6890N" von Agilent Technologies unter Verwendung einer DB-5-Säule (Länge: 20m, Innendurchmesser: 0,25 mm, Filmdicke 0,25 µm) von J&W Scientific und einem Flammenionisationsdetektor bei folgenden Rahmenbedingungen:

| | |
|---|---|
| Starttemperatur Ofen: 150 °C | Endtemperatur Ofen: 350 °C |
| (1) Heizrate 150-300 °C: 10 K/min | (2) Isotherm : 10 min. bei 300 °C |
| (3) Heizrate 300-350 °C: 25 K/min. | |
| Gesamtlaufzeit: 27 min. | |

| | |
|---|---|
| Eingangstemperatur Einspritzblock: 300 °C | Splittverhältnis: 200:1 |
| Splitfluss: 121,1 ml/min | Gesamtfluß: 124,6 ml/min. |
| Trägergas: Helium | Injektionsvolumen: 3 Mikroliter |

| | |
|---|---|
| Detektortemperatur: 350 °C | Brenngas: Wasserstoff |
| Flußrate Wasserstoff: 40 ml/min. | Flußrate Luft: 440 ml/min. |
| Makeup-Gas: Helium | Flußrate Makeup-Gas: 45 ml/min. |

Die Auswertung der erhaltenen Gaschromatogramme erfolgt manuell gegen vorhandene Vergleichssubstanzen die Angabe der Reinheit erfolgt in Flächenprozent. Auf Grund der hohen Endgehalte an Zielsubstanz von > 99,4 % ist der zu erwartende Fehler durch fehlende Kalibrierung auf die jeweilige Probensubstanz gering.

### 3. Bestimmung der APHA-Farbzahl

Die Bestimmung der Farbzahl der hergestellten Ester erfolgte gemäß DIN EN ISO 6271-2.

### 4. Bestimmung der Dichte

Die Bestimmung der Dichte der hergestellten Ester erfolgt mittels Biegeschwinger gemäß DIN 51757 - Verfahren 4.

### 5. Bestimmung der Säurezahl

Die Bestimmung der Säurezahl der hergestellten Ester erfolgte gemäß DIN EN ISO 2114.

### 6. Bestimmung des Wassergehaltes

Die Bestimmung des Wassergehaltes der hergestellten Ester erfolgte gemäß DIN 51777 Teil 1 (Direktes Verfahren).

### 7. Bestimmung der Eigenviskosität der Carbonsäureester

Die Bestimmung der Eigenviskosität (Scherviskosität) der hergestellten Ester erfolgte unter Verwendung eines Physia MCR 101 (Fa. Anton-Paar) mit Z3-Meßsystem (DIN 25mm) im Rotationsmodus über folgendes Verfahren:
Ester und Meßsystem wurden zunächst auf eine Temperatur von 20 °C temperiert, anschließend wurden folgende Punkte angesteuert:
   1. Eine Vorscherung von 100 s⁻¹ für den Zeitraum von 60 s, bei der keine Messwerte aufgenommen wurden (zur Nivellierung eventuell auftretender thixotroper Effekte und zur besseren Temperaturverteilung).
   2. Eine Frequenz-Abwärtsrampe, beginnend bei 500 s⁻¹ und endend bei 0,1 s⁻¹, aufgeteilt in eine logarithmische Reihe mit 20 Schritten mit jeweils 5 s Messpunktdauer (Verifizierung des newtonschen Verhaltens).

Alle Ester zeigten ein newtonsches Fließverhalten. Die Angabe der Viskositätswerte erfolgte exemplarisch bei einer Schergeschwindigkeit von 1 s⁻¹.

### 8. Bestimmung des Massenverlustes nach 10 Minuten bei 200 °C

Die Bestimmung des Massenverlustes der hergestellten Ester bei 200 °C erfolgte mit Hilfe eines Halogentrockners vom Typ "HB43S" (Fa. Mettler). Folgende Messparameter wurden eingestellt:
Temperaturrampe: konstant 200 °C
Messwertaufzeichnung: 30 s
Messdauer: 10 min
Probenmenge: 5 g

Für die Messung wurden Einwegschalen aus Aluminium (Fa. Mettler) und HS 1 Fiberfilter (Glasvlies Fa. Mettler) verwendet. Nach Nivellierung und Tarierung der Waage wurden die Proben (5 g) gleichmäßig auf dem Fiberfilter verteilt und die Messung gestartet. Für jede Probe wurde eine Doppelbestimmung durchgeführt, die Messwerte wurden gemittelt. Der letzte Messwert nach 10 min wird als "Masseverlust nach 10 Minuten bei 200 °C" angegeben.

### 9. Durchführung und Auswertung der Thermischen Analytik (DSC und TGA)

Die Bestimmung der Schmelzenthalpie und der Glasübergangstemperatur erfolgt über Differentialkalorimetrie (DSC) gemäß DIN 51007 (Temperaturbereich von - 100 °C bis + 200 °C) aus der ersten Aufheizkurve bei einer Heizrate von 10K/min. Der Wendepunkt der Wärmestromkurve wird als Glasübergangstemperatur ausgewertet. Die Bestimmung der Schmelzenthalpie erfolgt durch Integration der Peakfläche(n).

Die Durchführung der thermogravimetrischen Messung (TGA) erfolgte gemäß DIN 51006 (Temperaturbereich: 25 °C bis 310 °C) bei einer Heizrate von 10 K/min.

### 10. Bestimmung der Plastisolviskosität

Die Messung der Viskosität der PVC-Plastisole wurde mit einem Physica MCR 101 (Fa. Anton-Paar) durchgeführt, wobei der Rotationsmodus und das Meßsystem "Z3" (DIN 25 mm) verwendet wurden.

Das Plastisol wurde zunächst im Ansatzbehälter mit einem Spatel manuell homogenisiert, anschließend in das Messsystem eingefüllt und isotherm bei 25 °C vermessen. Während der Messung wurden folgende Punkte angesteuert:
1. Eine Vorscherung von 100 s⁻¹ für den Zeitraum von 60 s, bei der keine Messwerte aufgenommen wurden (zur Nivellierung eventuell auftretender thixotroper Effekte).
2. Eine Frequenz-Abwärtsrampe, beginnend bei 200 s⁻¹ und endend bei 0,1 s⁻¹, aufgeteilt in eine logarithmische Reihe mit 30 Schritten mit jeweils 5 Sekunden Messpunktdauer.

Die Messungen wurden in der Regel (wenn nicht anders angegeben) nach einer Lagerung / Reifung der Plastisole von 24h (bei 25 °C) durchgeführt.

### 11. Bestimmung der Geliergeschwindigkeit

Die Untersuchung des Gelierverhaltens der Plastisole wurde im Physica MCR 101 im Oszillationsmodus mit einem Platte-Platte Meßsystem (PP25), welches schubspannungsgesteuert betrieben wurde, vorgenommen. Eine zusätzliche Temperrierhaube wurde an das Gerät angeschlossen, um die bestmögliche Wärmeverteilung zu erreichen.

**Meßparameter:**

| | |
|---|---|
| Modus: | Temperatur-Gradient (Temperaturrampe) |
| | Start-Temperatur: 25 °C |
| | End-Temperatur: 180 °C |
| | Heiz/Kühlrate: 5 K/min |
| | Oszillations-Frequenz: 4 - 0,1 Hz Rampe (logarithmisch) |
| | Kreisfrequenz Omega: 10 1/s |
| | Anzahl Messpunkte: 63 |
| | Messpunktdauer: 0,5 min |
| | Automatische Spaltnachführung F : 0 N |
| | konstante Messpunktdauer |
| | Spaltweite 0,5 mm |

### Durchführung der Messung:

Auf die untere Messsystemplatte wurde ein Tropfen der zu messenden Plastisolrezeptur luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als ca. 6 mm rundum). Anschließend wurde die Temperierhaube über der Probe positioniert und die Messung gestartet.

Bestimmt wurde die sog. komplexe Viskosität des Plastisols in Abhängigkeit von der Temperatur. Ein Einsetzen des Geliervorganges war in einem plötzlichen starken Anstieg der komplexen Viskosität zu erkennen. Je früher dieser Viskositätsanstieg einsetzte, desto besser war die Gelierfähigkeit des Systems.

Aus den erhaltenen Meßkurven wurden durch Interpolation für jedes Plastisol die Temperaturen bestimmt, bei der eine komplexe Viskosität von 1000 Pa _{*} s bzw. 10.000 Pa*s erreicht war. Zusätzlich wurde mittels Tangentenmethode die im vorliegenden Versuchsaufbau maximal erreichte Plastisolviskosität bestimmt, sowie durch Fällen eines Lotes die Temperatur, ab der die maximale Plastisolviskosität auftritt.

### 12. Bestimmung des Gelbwertes an Schaum- und Deckstrichfolien

Der Gelbwert (Index YD 1925) ist ein Maß für Gelbverfärbung eines Probekörpers. Die Farbmessung erfolgte mit einem "Spectro Guide" Gerät der Fa. Byk-Gardner. Als Hintergrund für die Farbmessungen wurde eine weiße Referenz-Kachel benutzt. Folgende Parameter wurden eingestellt:
Lichtart: C/2°
Anzahl Messungen: 3
Anzeige: CIE L*a*b*
Messindex: YD1925

Die Messungen selbst wurden an 3 unterschiedlichen Stellen der Proben (für Effekt- und Glattschäume bei einer Plastisol-Rakeldicke von 200µm) durchgeführt. Die Werte aus den 3 Messungen wurde gemittelt.

### 13. Bestimmung der Shore Härte (Weichmachereffizienz)

Die Härte-Messungen wurden nach DIN 53 505 mit einem Shore-A-Messgerät der Fa. Zwick-Roell durchgeführt, der Messwert jeweils nach 3 Sekunden abgelesen. An jedem Probekörper (z.B. Gießling) wurden Messungen an drei verschiedenen Stellen durchgeführt, und ein Mittelwert gebildet.

### 14. Bestimmung der Opazität von Deckstrichfolien

Die Bestimmung der Opazität erfolgte mit einem "Spectro Guide" Gerät der Fa. Byk Gardner. Als Hintergrund für die Opazitätsmessungen wurden eine weiße Kachel und eine schwarze Kachel benutzt. Über das Menü am Farbmessgerät wurde die Opazitätsmessung angewählt. Die Messungen selbst wurden an 3 unterschiedlichen Stellen der Proben durchgeführt und automatisch ausgewertet.

### Beispiel 1:

### Analyse des zur Herstellung der Bernsteinsäureester verwendeten Isononylalkohols hinsichtlich des Gehaltes an 3,5,5-Trimethylhexanol und n-Nonanol

Die Ermittlung der Isomerenverteilung erfolgte mittels GC über die unter Analytik, Punkt 1 beschrieben Methode. Die Ergebnisse sind in Tabelle 1 zusammengefasst.

**Tabelle 1: Isomerenverteilungen**

| | 3,5,5-Trimethylhexanol [Ma%] | n-Nonanol [Ma%] | Alkohole mit mehr als 9 Kohlenstoffatomen [Ma%] | Alkohole mit weniger als 9 Kohlenstoffatomen [Ma%] | Anteil an Alkoholen mit 1 Methylverzweigung am β-C-Atom [Ma%] | Anteil an Alkoholen mit 2 Methylverzweigung am β-C-Atom [Ma%] |
|---|---|---|---|---|---|---|
| VESTINOL 9 | 0,03 | 7,6 | < 1 | < 1 | 15,8 | < 1 |
| Exxal 9 | 0,11 | 1,3 | 24,8 | 2,5 | 18,67 | < 1 |
| Exxal 9 S | 0,13 | 1,5 | 4,7 | 1,6 | 18,85 | < 1 |

### Beispiel 2:

### Herstellung der Carbonsäureester

### 2.1 Herstellung von Diisononyladipat (DINA) aus Adipinsäure und Isononanol der Firma EvoniK Oxeno GmbH (Vergleichsversuch)

In einem 4-Liter Rührkolben mit Wasserauskreiser, aufgesetztem Intensivkühler, Rührer, Tauchrohr, Tropftrichter und Thermometer, wurden 730 g (5 Mol) Adipinsäure (Fa. Sigma Aldrich), 0,44 g (0,06 Massen-% bezogen auf Adipinsäure) Tetrabutyl-orthotitanat (Vertec TNBT, Fa. Johnson Matthey Catalysts) und 1872 g (13 Mol) eines über den OCTOL-Prozess hergestellten Isononanols (Fa. Evonik Oxeno GmbH) vorgelegt, und bis 240 °C verestert. Nach 3 Stunden war die Reaktion beendet. Danach wurde bis 180 °C und 3 mbar der überschüssige Alkohol abdestilliert. Anschließend wurde auf 80 °C abgekühlt und mit 17 ml einer 10 Massen-%igen wässrigen NaOH-Lösung neutralisiert. Im Anschluss wurde bei einer Temperatur von 180 °C und einem Druck zwischen 20 und 5 mbar eine Wasserdampfdestillation durchgeführt. Danach wurde der Ansatz auf 130 °C abgekühlt und bei dieser Temperatur bei 5 mbar getrocknet. Nach Abkühlen auf <100 °C wurde der Ansatz über Filterhilfsmittel filtriert. Laut GC ergab sich ein Estergehalt (Reinheit) von > 99,9 %.

### 2.2 Herstellung von Di-2-Ethylhexylsuccinat (D2EHS) aus Bernsteinsäure und 2-Ethylhexanol (Vergleichsversuch)

In einem 4-Liter Rührkolben mit Wasserauskreiser, aufgesetztem Intensivkühler, Rührer, Tauchrohr, Tropftrichter und Thermometer wurden 826 g (7 Mol) Bernsteinsäure (Fa. Sigma Aldrich), 2,07 g (0,25 Massen-% bezogen auf Bernsteinsäure) Tetrabutyl-orthotitanat (Vertec TNBT, Fa. Johnson Matthey Catalysts) und 2210 g (17 Mol) 2-Ethylhexanol (Fa. Sigma Aldrich) vorgelegt, und bis 220 °C verestert. Nach 3 Stunden war die Reaktion beendet. Danach wurde bis 180 °C und 3 mbar der überschüssige Alkohol abdestilliert. Anschließend wurde auf 80 °C abgekühlt und mit 7,5 ml einer 10 Massen-%igen wässrigen NaOH-Lösung neutralisiert. Im Anschluss wurde der Ansatz bei einer Temperatur von 140 °C und einem Druck von 40 mbar durch durchleiten von Stickstoff gereinigt. Sodann wurde der Ansatz auf 90°C abgekühlt und durch Zugabe von 11,5 g (0,5% bezogen auf die verbliebene Estermenge) Aktivkohle (CAP Super der Fa. Norit) bei dieser Temperatur aufgehellt. Anschließend wurde der Ansatz bei < 90°C über Filterhilfsmittel (Perlite) filtriert. Laut GC ergab sich ein Estergehalt (Reinheit) von 99,46 %.

### 2.3 Herstellung von Di-Isononylsuccinat (DINS) aus Bernsteinsäure und Isononanol der Firma Evonik Oxeno GmbH (erfindungsgemäß)

In einem 4-Liter Rührkolben mit Wasserauskreiser, aufgesetztem Intensivkühler, Rührer, Tauchrohr, Tropftrichter und Thermometer wurden 826 g (7 Mol) Bernsteinsäure (Fa. Sigma Aldrich), 2,07 g (0,25 Massen-% bezogen auf Bernsteinsäure) Tetrabutyl-orthotitanat (Vertec TNBT, Fa. Johnson Matthey Catalysts) und 2448 g (17 Mol) eines über den OCTOL-Prozess hergestellten Isononanols (Fa. Evonik Oxeno GmbH) vorgelegt und bis 220 °C verestert. Nach 3 Stunden war die Reaktion beendet. Danach wurde bei 180 °C und 3 mbar der überschüssige Alkohol abdestilliert. Anschließend wurde auf 80 °C abgekühlt und mit 2 ml einer 10 Massen-%igen wässrigen NaOH-Lösung neutralisiert. Im Anschluss wurde der Ansatz bei einer Temperatur von 160 °C und einem Druck von 40 mbar durch durchleiten von Stickstoff gereinigt. Danach wurde der Ansatz auf 90 °C abgekühlt und durch Zugabe von 11,5 g (0,5% bezogen auf die verbliebene Estermenge) Aktivkohle (CAP Super der Fa. Norit) bei dieser Temperatur aufgehellt. Anschließend wurde der Ansatz bei < 90°C über Filterhilfsmittel filtriert. Laut GC ergab sich ein Estergehalt (Reinheit) von 99,84 %.

Charakteristische Materialparameter zu den in Beispiel 2 erhaltenen Estern sind in Tabelle 2 zusammengestellt.

**Tabelle 2: Materialparameter der Carbonsäureester.**

| Produkt (gemäß Beispiel) | Rein heit (GC) [FI-%] | Verzweigungsgrad (NMR) [-] | APHA-Farbe [-] | Dichte [g/cm³] | Säurezahl [mg KOH/g ] | Wassergehalt [%] | Eigenviskosität [mPa*s] | Masseverlust TGA bis 300 °C [%] | Masseverlust nach 10 Minuten @200 °C [ma%] | DSC | |
|---|---|---|---|---|---|---|---|---|---|---|---|
| | | | | | | | | | | T_{g} [°C] | ΔH_{M} [J/g] |
| Di(isononyl)phthalat, VESTINOL® 9, Fa. Evonik Oxeno GmbH **(Vergleichsbeispiel)** | 99,95 | 1,29 | 5 | 0,9741 | 0,016 | 0,023 | 76 | 7,5 | 3,7 | - 86 | 0 |
| Di(isononyl)adipat gemäß Beispiel 2.1 **(Vergleichsbeispiel)** | 99,9 | n.b. | 8 | 0,9216 | 0,019 | 0,01 | 19 | 21,1 | 7,3 | -106 | 19,2 |
| Di(2-ethylhexyl)succinat gemäß Beispiel 2.2 **(Vergleichsbeispiel)** | 99,46 | n.b. | 4 | 0,932 | 0,03 | 0,02 | 13 | 34,8 | 28,9 | -103 | 0 |
| Di(isononyl)succinat gemäß Beispiel 2.3 **(erfindungsgemäß)** | 99,84 | n.b. | 18 | 0,9269 | 0,02 | 0,005 | 18 | 14,5 | 13,8 | - 104 | 0 |

| | | | | | | | | | | | |
|---|---|---|---|---|---|---|---|---|---|---|---|
| n.b. = nicht bestimmt | | | | | | | | | | | |

### Beispiel 3:

### Exemplarische Formulierungen enthaltend die erfindungsgemäßen Bernsteinsäureester

Im Folgenden werden sie erfindungsgemäßen Bernsteinsäureester in allgemeinen PVC-Formulierungen eingesetzt, um die Anwendungsbreite der erfindungsgemäßen Ester zu verdeutlichen. Die im nachfolgenden dargestellten Formulierungen können bzw. müssen vom Fachmann an die im jeweiligen Anwendungsbereich bestehenden spezifischen Verarbeitungs- und Gebrauchsanforderungen angepasst werden.

### 3.1 Transparent-Deckstrich (Fußbodenbelag)

| | |
|---|---|
| 64,5 | Gewichtsanteile Suspensions-PVC mit einem K-Wert (gemäß DIN EN ISO 1628-2) von 60-75 (z.B. VESTOLIT B 7021-Ultra) |
| 32,3 | Gewichtsanteile erfindungsgemäßer Bernsteinsäureester gemäß Beispiel 2.3 |
| 1,9 | Gewichtsanteile epoxidiertes Sojabohnenöl als (Co)Stabilisator (z.B. DRAPEX 39) |
| 1,3 | Gewichtsanteil Stabilisator (z.B. MARK C/Z 149) |

### Herstellung des Plastisols

Die Herstellung des Plastisols erfolgte mit einem Kreiss Dissolver VDKV30-3 (Fa. Niemann). Vor den festen Bestandteilen wurden die flüssigen Bestandteile der Rezeptur in einem Mischbecher eingewogen. Von Hand wurde die Mischung mit einem Salbenspatel so eingerührt, dass kein unbenetztes Pulver mehr vorhanden war. Der Mischbecher wurde dann in die Klemmvorrichtung des Dissolverrührers eingespannt. Mit der geeigneten Mischerscheibe (D: 50mm) wurde die Probe homogenisiert. Während der Homogenisierung wurde mit Hilfe einer Vakuumpumpe im Mischgefäß ein Vakuum erzeugt. Der Druck im Mischbehälter wurde mit einem Vakuummeter (DVR 2 Fa. Vakuubrand) kontrolliert. Es wurde ein Druck (abs.) von unter 10 mbar erreicht.

Des Weiteren wurde die Drehzahl von 330 U/min bis 2000 U/min erhöht, und so lange gerührt, bis die Temperatur an der Digitalanzeige des Thermofühlers 30 °C erreichte. Damit war sicher gestellt, dass die Homogenisierung des Plastisols bei einem definierten Energieeintrag erreicht wurde. Danach wurde das Plastisol für weitere 10 min bei einer Drehzahl von 330 U/min gerührt und entlüftet. Nach Fertigstellung des Plastisols wurde es sofort bei 25 °C temperiert.

### Herstellung der Folien

Die Herstellung der Folien erfolgt in der Regel nach einer Reifezeit von 24 Stunden (bei 25 °C). Für die Folienherstellung wurde am Rollrakel eines Mathis Labcoaters (Hersteller: Fa. W. Mathis AG) ein Rakelspalt von 1,40 mm eingestellt. Dieser wurde mit einer Fühlerblattlehre kontrolliert und ggf. nachgestellt. Die hergestellten Plastisole wurden auf ein in einem Rahmen plan eingespanntes Hochglanzpapier (Ultracast Patent; Fa. Sappi Ltd.) mittels dem Rollrakel des Mathis Labcoaters aufgerakelt. Das aufgerakelte Plastisol wurde nun für 2 min im Mathis Ofen bei 200°C ausgeliert. Nach Abkühlung wurde die Foliendicke mit Hilfe eines Dickenschnellmessers (KXL047; Fa. Mitutoyo) mit einer Genauigkeit von 0,01 mm bestimmt. Die Foliendicke dieser Folie betrug bei dem angegebenen Rakelspalt in allen Fällen zwischen 0,95 und 1,05 mm. Die Messung der Dicke wurde an drei unterschiedlichen Stellen der Folie durchgeführt.

### 3.2 Rückseitenschaum (Fußbodenbelag)

| | |
|---|---|
| 61,5 | Gewichtsanteile PVC mit einem K-Wert (gemäß DIN EN ISO 1628-2) von 65-75 (z.B. VINNOLIT MP 6852) |
| 36 | Gewichtsanteile erfindungsgemäßer Bernsteinsäureester gemäß Beispiel 2.3 |
| 1,5 | Gewichtsanteile thermisch aktivierbares Treibmittel (z.B. auf Basis Azodicarbonamid) |
| 1 | Gewichtsanteil Zinkoxid |

### Herstellung des Plastisols

Die Herstellung der Plastisols erfolgte wie in Beispiel 3.1 beschrieben jedoch unter Verwendung der in Beispiel 3.2 aufgeführten Rezeptur.

### Herstellung der Folien

Die Herstellung der Folien erfolgt in der Regel nach einer Plastisol-Reifezeit von 24 Stunden (bei 25 °C). Für die Folienherstellung wurde am Rollrakel eines Mathis Labcoaters (Hersteller: Fa. W. Mathis AG) ein Rakelspalt von 1,00 mm eingestellt. Dieser wurde mit einer Fühlerblattlehre kontrolliert und ggf. nachgestellt. Die hergestellten Plastisole wurden auf ein in einem Rahmen plan eingespanntes Trennpapier (Warren Release Paper - Stripkote EHR; Fa. Sappi Ltd.) mittels dem Rollrakel des Mathis Labcoaters aufgerakelt. Das aufgerakelte Plastisol wurde nun für 30 s im Mathis Ofen bei 200 °C angeliert. Nach Abkühlung wurde die Foliendicke mit Hilfe eines Dickenschnellmessers (KXL047; Fa. Mitutoyo) mit einer Genauigkeit von 0,01 mm bestimmt. Die Foliendicke dieser Folie betrug bei dem angegebenen Rakelspalt in allen Fällen zwischen 0,74 und 0,77 mm. Die Messung der Dicke wurde an drei unterschiedlichen Stellen der Folie durchgeführt. Anschließend wurden ebenfalls mit dem bzw. im Mathis-Labcoater die geschäumten Folien (Schäume) bei unterschiedlichen Ofen-Verweilzeiten (z.B. 60s, 90s, 120s und 150s) hergestellt.

### 3.3 Schaumschicht zur Beschichtung (Fußbodenbelag)

| | |
|---|---|
| 35 | Gewichtsanteile PVC mit einem K-Wert (gemäß DIN EN ISO 1628-2) von 65-75 (z.B. VESTOLIT P 1352 K) |
| 24,5 | Gewichtsanteile erfindungsgemäßer Bernsteinsäureester gemäß Beispiel 2.3 |
| 35 | Gewichtsanteile Calciumcarbonat (z.B. Calcilit 8 G) |
| 2,5 | Gewichtsanteile Titandioxid (z.B. Kronos 2220) |
| 1 | Gewichtsanteil thermisch aktivierbares Treibmittel (z.B. auf Basis Azodicarbonamid) |
| 1 | Gewichtsanteile Zinkoxid |
| 1 | Gewichtsanteil Isopropanol |

### Herstellung des Plastisols

Die Herstellung der Plastisols erfolgte wie in Beispiel 3.1 beschrieben jedoch unter Verwendung der in Beispiel 3.3 aufgeführten Rezeptur.

### Herstellung der Folie

Die Herstellung der Folien erfolgte wie in Beispiel 3.2 beschrieben jedoch unter Verwendung eines gemäß Beispiel 3.3 hergestellten Plastisols.

### 3.4 Fußbodenbelag kalandriert (Dryblend)

| | |
|---|---|
| 65 | Gewichtsanteile Suspensions-PVC mit einem K-Wert (gemäß DIN EN ISO 1628-2) von 65-75 (z.B. SolVin 271 PC) |
| 31 | Gewichtsanteile erfindungsgemäßer Bernsteinsäureester gemäß Beispiel 2.3 |
| 0,5 | Gewichtsanteile Stabilisator (z.B. metallfreier organischer Stabilisator wie Mark OBS 1100) |
| 1 | Gewichtsanteil Stabilisator (z.B. metallfreier organischer Stabilisator wie Mark OBS 1360) |
| 2 | Gewichtsanteile epoxidiertes Sojabohnenöl als (Co)Stabilisator (z.B. DRAPEX 39) |
| 0,5 | Gewichtsanteile Calciumstearat (als Prozeßhilfsmittel / Gleitmittel) |

### Herstellung der Dryblends

Die Herstellung der Dryblends erfolgte in einem Brabender Planetenmischer. Der Mischbehälter des Planetenmischers wurde auf eine konstante Temperatur von 90°C temperiert. Es wurden softwareseitig folgende Parameter am Planetenmischer eingestellt.
Drehzahlprogramm: ja
Profil: Drehzahl 50 U/min; Haltezeit 9min; Anstiegszeit 1 min
Drehzahl 100 U/min; Haltezeit 20 min
Knetertemperatur: 88 °C
Messbereich: 2 Nm
Dämpfung: 3

Durch auftretende Wärmeverluste erreichte die Temperatur im Mischbehälter 88°C. Nachdem der Planetenmischer eine Eigenkalibrierung durchgeführt hatte, wurden die festen Bestandteile über einen Trichter dem Mischgefäß zugeführt. Das Programm wurde gestartet und die Pulvermischung wurde 10 Minuten im Mischgefäß gerührt, ehe die flüssigen Bestandteile zugegeben wurden. Die flüssigen Bestandteile wurden vorgewogen. Die Mischung wurde nun weitere 20 Minuten im Planetenmischer gerührt. Nach Beendigung des Programms wurde der fertige Dryblend entnommen und bei Raumtemperatur. Das ü Drehmoment-Zeit Diagramm wurde über die Saftware ausgewertet. Nach der Zugabe der flüssigen Bestandteile ist ein deutlicher Kurvenanstieg zu erkennen. Erst wenn die Kurve wieder deutlich abfällt, ist die Weichmacheraufnahme abgeschlossen. Die Zeitdifferenz dieser beiden Punkte ist die Weichmacheraufnahmezeit (Dryblendzeit). Das maximale Drehmoment wird vom Programm automatisch ausgewertet.

### Verarbeitung der Dryblends

### Herstellung der Walzfelle

Die Herstellung der Walzfelle erfolgte auf einem Kalander W150 AP der Fa. Collin. Folgende Parameter wurden dazu am Kalander eingestellt:
Walzentemperatur: 165 °C
Walzenspalt: 0,5 mm
Walzzeit: 5 min
Fünfstufiges Programm zur Herstellung des Walzfelles

Nach dem Erreichen der Walzentemperatur wurde der Walzenspalt kalibriert. Zum Start der Messung wurde der Walzenspalt auf 0,2 mm eingestellt. Der Dryblend wurde in einem eingewogen (i.d.R. ca. 160 g Gesamtmasse) und bei stehenden Walzen in den Walzenspalt gegeben. Das Programm wurde gestartet. Die Walzen starteten mit einer Umdrehungszahl von 5 U/min und einer Friktion von 20%. Nach ca. 1 min war die Plastifizierung zum größten Teil abgeschlossen und der Walzenspalt wurde auf 0,5 mm vergrößert. Es erfolgte eine 3-malige Homogenisierung mittels automatischer Umlegeeinheit am Kalander. Nach 5 min wurde das Walzfell von der Walze entfernt und abgekühlt.

### Herstellung der Pressplatten

Die Pressplatten wurden an einer Laborpresse der Fa. Collin hergestellt. Die vorgefertigten Walzfelle (siehe oben) wurden zur Herstellung der Pressplatten verwendet. Die Seitenränder der Walzfelle wurden mit Hilfe einer Schneidemaschine entfernt, das Walzfell wurde anschließend in ca. 14,5 x 14,5 cm große Stücke geschnitten. Für 1 mm dicke Pressplatten wurden je 2 Walzfellstücke in den 15 x 15 cm großen Pressrahmen aus Edelstahl gelegt. Folgende Parameter wurden an der Laborpresse eingestellt:
Dreiphasiges Programm:
   Phase 1: Beide Platten 165°; Pressplattendruck: 5 bar; Phasenzeit: 60 Sekunden.
   Phase 2: Beide Platten 165°; Pressplattendruck: 200 bar; Phasenzeit: 120 Sekunden.
   Phase 3: Beide Platten 40°; Pressplattendruck: 200 bar; Phasenzeit: 270 Sekunden.

Die überschüssige Presslippe wurde nach Herstellung der Pressplatten entfernt.

### 3.5 Tapetenrezeptur Boucle-Schaum (Effektschaum)

| | |
|---|---|
| 48 | Gewichtsanteile PVC mit einem K-Wert (gemäß DIN EN ISO 1628-2) von 65-75 (z.B. VESTOLIT E 7012 S) |
| 26 | Gewichtsanteile erfindungsgemäßer Bernsteinsäureester gemäß Beispiel 2.3 |
| 2,5 | Gewichtsanteile thermisch aktivierbares Treibmittel (z.B. auf Basis Azodicarbonamid) |
| 15 | Gewichtsanteile Calciumcarbonat (z.B. Calcilit 8 G) |
| 4 | Gewichtsanteile Titandioxid (z.B. Kronos 2220) |
| 1,5 | Gewichtsanteile Zersetzungskatalysator / "Kicker" auf K/Zn-Basis (z. B. Baerostab KK 48) |
| 1,5 | Gewichtsanteile paraffinisches Lösungsmittel (z.B. Isopar J) |
| 1,5 | Gewichtsanteile Isopropanol |

### Herstellung der Plastisole

Die Herstellung der Plastisole erfolgte mit einem Laborrührer "Eurostar" (Fa. IKA). An dem Rührer wurde eine Zahnmischerscheibe mit einem Durchmesser von 50 mm montiert. Vor den festen Bestandteilen wurden die flüssigen Bestandteile der Rezeptur in einem PE Mischbecher auf einer Waage (Mettler XS6002S) eingewogen. Von Hand wurde die Mischung mit einem Salbenspatel so eingerührt, dass kein unbenetztes Pulver mehr vorhanden war. Der Laborrührer wurde dann in die Mischung getaucht und die Drehzahl des Rührers wurde innerhalb von 15 s von 0 auf 2000 U/min gesteigert. Für weitere 45 s wurde die Mischung bei der Drehzahl von 2000 U/min homogenisiert, so dass für alle Plastisole eine Mischzeit von 60 s eingehalten wurde. Nach Fertigstellung des Plastisols wurde es sofort bei 25,0 °C temperiert.

### Verarbeitung der Plastisole / Herstellung der Tapetenschäume

Nach einer Lagerzeit von mindestens zwei Stunden und maximal 24 Stunden wurden die Plastisole an einem Mathis Ofen (Typ LTE-TS) verschäumt. Als Träger wurde ein beschichtetes Tapetenpapier (Fa. Ahlstrom GmbH) gewählt. Mit einer Rakelbeschichtungseinheit wurden die Plastisole in 3 unterschiedlichen Stärken (300 µm, 200 µm und 100 µm) aufgetragen. Es wurden jeweils 3 Plastisole nebeneinander verstrichen. Bei 3 verschiedenen Temperaturen (200 °C, 210 °C und 220 °C) wurden die Plastisole bei einer Verweilzeit von 60 Sekunden ausgeschäumt. Nach Abkühlung der Schäume wurden die Tapeten für die weitere Bearbeitung zugeschnitten.

### 3.6 Tapetenrezeptur Glattschaum

| | |
|---|---|
| 18 | Gewichtsanteile PVC mit einem K-Wert (gemäß DIN EN ISO 1628-2) von 65-75 (z.B. VESTOLIT E 7012 S) |
| 16 | Gewichtsanteile Emulsions-PVC mit einem einem K-Wert (gemäß DIN EN ISO 1628-2) von 65-75 (z.B. VINNOLIT E 67 ST) |
| 13,5 | Gewichtsanteile Mikrosuspensions-PVC mit einem K-Wert (gemäß DIN EN ISO 1628-2) von 55-67 (z.B. VESTOLIT B 6021-Ultra) |
| 25 | Gewichtsanteile erfindungsgemäßer Bernsteinsäureester gemäß Beispiel 2.3 |
| 1,5 | Gewichtsanteile thermisch aktivierbares Treibmittel (z.B. auf Basis Azodicarbonamid) |
| 3 | Gewichtsanteile epoxidiertes Sojabohnenöl als (Co)Stabilisator (z.B. DRAPEX 39) |
| 2 | Gewichtsanteile Titandioxid (z.B. Kronos 2220) |
| 20 | Gewichtsanteile Calcium-Magnesiumcarbonat (z.B. Microdol A 1) |
| 1 | Gewichtsanteil Zersetzungskatalysator / "Kicker" auf K/Zn-Basis (z. B. Baerostab KK 48) |

### Herstellung des Plastisols

Die Herstellung der Plastisols erfolgte wie in Beispiel 3.5 beschrieben jedoch unter Verwendung der in Beispiel 3.6 aufgeführten Rezeptur.

### Herstellung der Folie

Die Herstellung der Tapeten erfolgte wie in Beispiel 3.5 beschrieben jedoch unter Verwendung eines gemäß Beispiel 3.6 hergestellten Plastisols.

### 3.7 Tapetenrezeptur Kompakt-Matt

| | |
|---|---|
| 28 | Gewichtsanteile PVC mit einem K-Wert (gemäß DIN EN ISO 1628-2) von 65-75 (z.B. VESTOLIT E 7012 S) |
| 26 | Gewichtsanteile erfindungsgemäßer Bernsteinsäureester gemäß Beispiel 2.3 |
| 37 | Gewichtsanteile gefälltes unbeschichtetes Calciumcarbonat (z.B. SOCAL N2R) |
| 4,5 | Gewichtsanteile Titandioxid (z.B. Kronos 2220) |
| 3 | Gewichtsanteile epoxidiertes Sojabohnenöl als (Co)Stabilisator (z.B. DRAPEX 39) |
| 1,5 | Gewichtsanteile Stabilisator (z.B. Mark B/Z 562) |

### Herstellung des Plastisols

Die Herstellung der Plastisols erfolgte wie in Beispiel 3.5 beschrieben jedoch unter Verwendung der in Beispiel 3.7 aufgeführten Rezeptur.

### Herstellung der Folie

Die Herstellung der Tapeten erfolgte wie in Beispiel 3.5 beschrieben jedoch unter Verwendung eines gemäß Beispiel 3.7 hergestellten Plastisols.

### 3.8 PVC-Planen Beschichtungsmasse

| | |
|---|---|
| 55 | Gewichtsanteile PVC mit einem K-Wert (gemäß DIN EN ISO 1628-2) von 65-75 (z.B. VESTOLIT P 1430 K70) |
| 33 | Gewichtsanteile erfindungsgemäßer Bernsteinsäureester gemäß Beispiel 2.3 |
| 8 | Gewichtsanteile Calciumcarbonat (z.B. Calcilit 8 G) |
| 1,5 | Gewichtsanteile Titandioxid (z.B. Kronos 2220) |
| 1,5 | Gewichtsanteile epoxidiertes Sojabohnenöl als (Co)Stabilisator (z.B. DRAPEX 39) |
| 1 | Gewichtsanteil Stabilisator (z.B. MARK B/Z 561) |

### Herstellung des Plastisols

Die Herstellung der Plastisols erfolgte wie in Beispiel 3.1 beschrieben jedoch unter Verwendung der in Beispiel 3.8 aufgeführten Rezeptur.

### Herstellung der Folie

Die Herstellung der Planen erfolgte wie in Beispiel 3.1 beschrieben jedoch unter Verwendung eines gemäß Beispiel 3.8 hergestellten Plastisols.

### 3.9 PVC-Dachbahn

| | |
|---|---|
| 65 | Gewichtsanteile Suspensions-PVC mit einem K-Wert (gemäß DIN EN ISO 1628-2) von 65-75 (z. B. SolVin 271 PC) |
| 31,5 | Gewichtsanteile erfindungsgemäßer Bernsteinsäureester gemäß Beispiel 2.3 |
| 2 | Gewichtsanteile epoxidiertes Sojabohnenöl als (Co)Stabilisator (z.B. DRAPEX 39) |
| 1 | Gewichtsanteil Stabilisator (z.B. MARK B/Z 561) |
| 0,5 | Gewichtsanteile Calciumstearat |

### Herstellung des Dryblends

Die Herstellung der Dryblends erfolgte wie in Beispiel 3.4 beschrieben jedoch unter Verwendung der in Beispiel 3.9 aufgeführten Rezeptur.

### Herstellung der Pressplatten

Die Herstellung der Pressplatten erfolgte wie in Beispiel 3.4 beschrieben jedoch unter Verwendung eines gemäß Beispiel 3.9 hergestellten Dryblends.

### 3.10 PVC-Schlauchrezeptur (gefüllt)

| | |
|---|---|
| 65 | Gewichtsanteile Suspensions-PVC mit einem K-Wert (gemäß DIN EN ISO 1628-2) von 65-75 (z. B. SolVin 271 PC) |
| 20 | Gewichtsanteile erfindungsgemäßer Bernsteinsäureester gemäß Beispiel 2.3 |
| 12,5 | Gewichtsanteile Calciumcarbonat (z.B. Omya EXH1-OM) |
| 1,5 | Gewichtsanteile Titandioxid (z.B. Kronos 2220) |
| 0,5 | Gewichtsanteile Stabilisator (z.B. Baerostab MC 8763-1 CP) |
| 0,5 | Gewichtsanteile Gleitmittel auf Basis Fettsäureester (z.B. Loxiol G 40) |

### Herstellung des Dryblends

Die Herstellung der Dryblends erfolgte wie in Beispiel 3.4 beschrieben jedoch unter Verwendung der in Beispiel 3.10 aufgeführten Rezeptur.

### Herstellung der Pressplatten

Die Herstellung der Pressplatten erfolgte wie in Beispiel 3.4 beschrieben jedoch unter Verwendung eines gemäß Beispiel 3.10 hergestellten Dryblends.

### 3.11 Kabelummantelung

| | |
|---|---|
| 46,5 | Gewichtsanteile Suspensions-PVC mit einem K-Wert (gemäß DIN EN ISO 1628-2) von 65-75 (z.B. SolVin 271 PC) |
| 29 | Gewichtsanteile erfindungsgemäßer Bernsteinsäureester gemäß Beispiel 2.3 |
| 23 | Gewichtsanteile Calciumcarbonat (z.B. OMYA BSH) |
| 1,5 | Gewichtsanteile Stabilisator (z.B. Baeropan MC KA 83/5) |

### Herstellung des Dryblends

Die Herstellung der Dryblends erfolgte wie in Beispiel 3.4 beschrieben jedoch unter Verwendung der in Beispiel 3.11 aufgeführten Rezeptur.

### Herstellung der Pressplatten

Die Herstellung der Pressplatten erfolgte wie in Beispiel 3.4 beschrieben jedoch unter Verwendung eines gemäß Beispiel 3.11 hergestellten Dryblends.

### 3.12 Kabelisoliermasse

| | |
|---|---|
| 43 | Gewichtsanteile Suspensions-PVC mit einem K-Wert (gemäß DIN EN ISO 1628-2) von 65-75 (z. B. SolVin 271 PC) |
| 21 | Gewichtsanteile erfindungsgemäßer Bernsteinsäureester gemäß Beispiel 2.3 |
| 34 | Gewichtsanteile Calciumcarbonat (z.B. OMYA BSH) |
| 2 | Gewichtsanteile Stabilisator (z.B. Baeropan MC KA 83/5) |

### Herstellung des Dryblends

Die Herstellung der Dryblends erfolgte wie in Beispiel 3.4 beschrieben jedoch unter Verwendung der in Beispiel 3.12 aufgeführten Rezeptur.

### Herstellung der Pressplatten

Die Herstellung der Pressplatten erfolgte wie in Beispiel 3.4 beschrieben jedoch unter Verwendung eines gemäß Beispiel 3.12 hergestellten Dryblends.

### 3.13 Schutzmasse (UBS)

| | |
|---|---|
| 32 | Gewichtsanteile Suspensions-PVC mit einem K-Wert (gemäß DIN EN ISO 1628-2) von 65-75 (z.B. VESTOLIT E7031) |
| 41 | Gewichtsanteile erfindungsgemäßer Bernsteinsäureester gemäß Beispiel 2.3 |
| 21,5 | Gewichtsanteile beschichtetes Calciumcarbonat (z.B. SOCAL 312) |
| 2 | Gewichtsanteile (Weiß) Feinkalk/Branntkalk (z.B. PRECAL 30S) |
| 1 | Gewichtsanteil Haftvermittler (z.B. Nouribond 323; Fa. Air Products) |
| 0,5 | Gewichtsanteile Zinkoxid (z.B. Zinkoxid Aktiv) |
| 2 | Gewichtsanteile aliphatisches Lösungsmittel mit Siedepunkt > 180 °C (z.B. Shellsol D70) |

### Herstellung des Plastisols

Die Herstellung der Plastisols erfolgte wie in Beispiel 3.1 beschrieben jedoch unter Verwendung der in Beispiel 3.13 aufgeführten Rezeptur.

Im Folgenden werden die erfindungsgemäßen Bernsteinsäureester in ausgewählten PVC-Formulierungen eingesetzt, und umfänglich hinsichtlich der Material-, Verarbeitungs- und Produkteigenschaften untersucht, um die erhältlichen Vorteile im Detail aufzuzeigen. Die im nachfolgenden dargestellten Formulierungen sind dabei ebenfalls allgemein gehalten, und können / müssen vom Fachmann an die im jeweiligen Anwendungsbereich bestehenden spezifischen Verarbeitungs- und Gebrauchsanforderungen angepasst werden.

### Beispiel 4:

### Verwendung von Bernsteinsäurediisononylestern (Diisononylsuccinaten) in PVC-Deckstrichformulierung (Plastisol) - Herstellung der Deckstrich-Plastisole .

Die nachfolgende Formulierung ist beispielhaft für die Verwendung der erfindungsgemäßen Bernsteinsäureester in Transparenten Deckstrichschichten, wie sie z.B. bei der Herstellung von (mehrlagigen) PVC-Fußbodenbelägen eingesetzt werden. Die Herstellung der Plastisole erfolgte gemäß Beispiel 3.1 jedoch mit veränderter Rezeptur. Die verwendeten Einwaagen der Komponenten für die verschiedenen Plastisole sind der nachfolgenden Tabelle (3) zu entnehmen.

**Tabelle 3: PVC-Deckstrichrezepturen.**

| [Alle Angaben in phr (= Massenteile pro 100 Massenteile PVC)] | | | | |
|---|---|---|---|---|
| **Plastisolrezeptur** | **1**** | **2**** | **3**** | **4*** |
| Vestolit B 7021 --Ultra | 100 | 100 | 100 | 100 |
| VESTINOL® 9 | 50 | | | |
| Di(isononyl)adipat gemäß Bsp. 2.1 | | 50 | | |
| Di(2-ethylhexyl)succinat gemäß Bsp. 2.2 | | | 50 | |
| Di(isononyl)succinat gemäß Bsp. 2.3 | | | | 50 |
| Drapex 39 | 3 | 3 | 3 | 3 |
| Mark CZ 149 | 2 | 2 | 2 | 2 |

| | | | | |
|---|---|---|---|---|
| ** = Vergleichsbeispiel * = Erfindungsgemäß | | | | |

Die verwendeten Stoffe und Substanzen werden im Folgenden näher erläutert:
**Vestolit B 7021-Ultra:** Mikrosuspensions-PVC (Homopolymer) mit einem K-Wert (bestimmt gemäß DIN EN ISO 1628-2) von 70; Fa. Vestolit GmbH.
**VESTINOL®** 9: Diisononyl(ortho)phthalat (DINP), Weichmacher; Fa. Evonik Oxeno GmbH.
**Drapex 39:** Epoxidiertes Sojabohnanöl; Costabilisator mit weichmachender Wirkung; Fa. Chemtura / Galata Chemicals.
**Mark CZ 149:** Calcium/Zink-Stabilisator; , Fa. Chemtura / Galata Chemicals.

### Beispiel 5:

### Bestimmung der Plastisolviskosität der Deckstrichplastisole enthaltend Diisononylsuccinate nach einer Lagerdauer von 24 h (bei 25 °C).

Die Messung der Viskositäten der in Beispiel 4 hergestellten Plastisole erfolgte mit einem Rheometer Physica MCR 101 (Fa. Paar-Physica), gemäß der unter Analytik, Punkt 11 beschriebenen Vorgehensweise. Die Ergebnisse werden in der nachfolgenden Tabelle (4) exemplarisch für die Schergeschwindigkeiten 100/s, 10/s, 1/s und 0,1/s dargestellt.

**Tabelle 4: Scherviskosität der Plastisole aus Beispiel 4 nach 24h Lagerung bei 25 °C.**

| **Plastisolrezeptur gemäß Bsp. 4** | **1**** | **2**** | **3**** | **4*** |
|---|---|---|---|---|
| Scherviskosität bei Schergeschwindigkeit= 100/s [Pa*s] | 6,8 | 1 | 0,7 | 1 |
| Scherviskosität bei Schergeschwindigkeit= 10/s [Pa*s] | 3,2 | 0,63 | 0,46 | 0,58 |
| Scherviskosität bei Schergeschwindigkeit= 1/s [Pa*s] | 2,8 | 0,65 | 0,48 | 0,58 |
| Scherviskosität bei Schergeschwindigkeit= 0,1/s [Pa*s] | 3,19 | 0,82 | 0,63 | 0,73 |

| | | | | |
|---|---|---|---|---|
| ** = Vergleichsbeispiel * = Erfindungsgemäß n.bb.= nicht bestimmbar | | | | |

Im Vergleich zum DINP-Standardplastisol (1) weisen alle anderen Plastisole eine deutlich niedrigere Scherviskosität auf, wobei diese -wie zu erwarten- beim erfindungsgemäßen Plastisol (4) leicht höher liegt als beim analogen Plastisol auf Basis von Di(2-ethylhexyl)succinat. Es werden somit erfindungsgemäße Plastisole zur Verfügung gestellt, die eine sehr niedrige Plastisolviskosität aufweisen und dadurch eine deutlich bessere Verarbeitbarkeit besitzen als die bekannten DINP-Plastisole. Im Vergleich mit den ebenfalls bekannten Plastisolen auf Basis Di(isononyladipat) sind die einfachere Zugänglichkeit aus nachwachsenden Rohstoffen sowie der Preisvorteil Bernsteinsäure gegenüber Adipinsäure als Vorteil für die erfindungsgemäßen Plastisole zu nennen. Die deutlich geringere Plastisolviskosität bietet dem Fachmann zudem die Möglichkeit durch entsprechende Rezepturanpassungen die Gesamtweichmachermenge deutlich zu reduzieren.

### Beispiel 6 :

### Bestimmung des Gelierverhaltens der in Beispiel 4 hergestellten PVC-Deckstrich-Plastisole.

Die Untersuchung des Gelierverhaltens der in Beispiel 4 hergestellten PVC-Deckstrich-Plastisole erfolgte wie unter Analytik, Punkt 12. (siehe oben) beschrieben, mit einem Physica MCR 101 im Oszillationsmodus nach einer Lagerung der Plastisole bei 25 °C für 24 h. Die Ergebnisse werden in der nachfolgenden Tabelle (5) dargestellt.

**Tabelle 5: Aus den Gelierkurven (Viskositätskurven) bestimmte Eckpunkte des Gelierverhaltens der gemäß Beispiel 4 hergestellten PVC-Deckstrich-Plastisole.**

| **Plastisolrezeptur (gemäß Bsp. 4)** | **1**** | **2**** | **3**** | **4*** |
|---|---|---|---|---|
| Erreichen einer Plastisolviskosität von 1.000 Pa*s bei [°C] | 89 | 123 | 101 | 122 |
| Erreichen einer Plastisolviskosität von 10.000 Pa*s bei [°C] | 103 | 139 | 126 | 140 |
| Maximale Plastisolviskosität [Pa*s] | 29.300 | 13.500 | 20.600 | 13.700 |
| Temperatur beim Erreichen der maximalen Plastisolviskosität [°C] | 137 | 146 | 137 | 145 |

| | | | | |
|---|---|---|---|---|
| ** = Vergleichsbeispiel * = Erfindungsgemäß | | | | |

Die Gelierung des erfindungsgemäßen Plastisols verläuft deutlich langsamer als die des DINP-Standardplastisols jedoch fast Deckungsgleich mit dem auf Di(isononyl)adipat basierenden Plastiol. Das Plastisol, welches auf Di(2-ethylhexyl)succinat basiert, nimmt eine Mittelstellung ein. Die langsamere Gelierung bedeutet andererseits, dass auch bei höheren Temperaturen noch eine reversible formgebende Verarbeitung der Plastisole ohne Verfestigung möglich ist. Das Defizit in der Geliergeschwindigkeit gegenüber den DINP-Standardplastisolen kann bei Bedarf vom Fachmann einfach durch die Zugabe von anderen Weichmachern, insbesondere von schnell gelierenden Weichmachern (z.B. Terephthalate mit C₄ oder C₅-Esterketten bzw. Benzoate mit C₅ bis C₁₃-Estergruppen) ausgeglichen werden.

### Beispiel 7 :

### Bestimmung der weichmachenden Wirkung bzw. der Weichmachereffizienz an Gießlingen durch Bestimmung der Shore-Härte (Shore A)

Die Shore-Härte ist ein Maß für die Weichheit eines Probekörpers. Je weiter bei einer bestimmten Messdauer eine genormte Nadel in den Probenkörper eindringen kann, desto niedriger fällt der Messwert aus. Der Weichmacher mit der höchsten Effizienz ergibt bei gleicher Weichmachermenge den niedrigsten Wert für die Shore Härte. Da in der Praxis Formulierungen / Rezepturen häufig auf eine bestimmte Shorehärte hin eingestellt bzw. optimiert werden, kann bei sehr effizienten Weichmachern demnach ein bestimmter Anteil in der Rezeptur eingespart werden, was eine Kostenreduktion für den Verarbeiter bedeutet. Zur Bestimmung der Shore-Härten wurden die gemäß Beispiel 4 hergestellten Plastisole in runde Gießformen aus Messing mit einem Durchmesser von 42 mm gegossen (Einwaage: 20,0 g). Dann wurden die Plastisole in den Formen im Umlufttrockenschrank 30 min bei 200 °C geliert, nach Abkühlung entnommen und vor der Messung mindestens 24 Stunden im Trockenschrank (25 °C) gelagert. Die Dicke der Scheiben betrug ca. 12 mm Die Ergebnisse der Härtebestimmung sind in Tabelle 6 zusammengestellt.

**Tabelle 6: Härte nach Shore A an Gießlingen hergestellt (gemäß Beispiel 7) aus den die erfindungsgemäßen Diisononylsuccinate enthaltenden Deckstrichplastisolen (gemäß Beispiel 4).**

| Plastisolrezeptur gemäß Bsp. 4 | **1**** | **2**** | **3**** | **4*** |
|---|---|---|---|---|
| Shore A | 80 | 79 | 75 | 80 |

| | | | | |
|---|---|---|---|---|
| ** = Vergleichsbeispie * = Erfindungsgemäß | | | | |

Die erfindungsgemäßen Di(isononyl)succinate weisen die gleiche Weichmachereffizienz (bezogen auf die Weichmachermasse) auf, wie das bekannte DINP (= Standardweichmacher). Es werden somit erfindungsgemäße Ester zur Verfügung gestellt, die bei deutlich niedrigerer Eigenviskosität eine dem DINP-Standard vergleichbare weichmachende Wirkung aufweisen, jedoch phthalatfrei sind und auf Basis nachwachsender Rohstoffe hergestellt werden können.

### Beispiel 8:

### Bestimmung von Opazität, Gelbwert und Ausschwitzverhalten der Deckstrichfolien.

Die Herstellung der Deckstrichfolien erfolgte wie in Beispiel 3.1 beschrieben, jedoch unter Verwendung der Plastisole aus Beispiel 4.
Die Transparenz ist ein wesentliches Kriterium zur Qualitätsbeurteilung von PVC-Deckstrichen im Fußbodenbereich, da nur bei hoher Transparenz (= geringer Opazität) ein optimales Gesamterscheinungsbild erzielt werden kann. Die Transparenz einer PVC-Deckstrichfolie gilt auch als Maß für die Verträglichkeit der zur Folienherstellung verwendeten Rezepturbestandteile, insbesondere als Maß zur Bewertung der Verträglichkeit von PVC-Matrix und Weichmacher. Hohe Transparenz (= geringer Opazität) bedeutet in der Regel eine gute Verträglichkeit. Die Bestimmung der Opazität erfolgte wie unter Analytik, Punkt 14 beschrieben, unter Verwendung der in Beispiel 8 hergestellten Deckstrichfolien. Der Gelbwert ist ein weiters wichtiges Qualitätskriterium. Eine Gelbfärbung im Deckstrich kann zu einer erheblichen optischen Beeinträchtigung eines Fußbodendekors führen, weshalb beim PVC-Deckstrich in der Regel nur sehr geringe Gelbwerte tolleriert werden können. Die Gelbverfärbung kann einerseits durch Rezepturbestandteile (wie auch durch ihre Neben- und Abbauprodukte) hervorgerufen werden, andererseits durch (z.B. thermooxidativen) Abbau während des Herstellprozesses und/oder der Verwendung des Deckstriches bzw. des Fußbodenbelages auftreten. Die Bestimmung des Gelbwertes erfolgte wie unter Analytik, Punkt 12 beschrieben, unter Verwendung der in Beispiel 8 hergestellten Deckstrichfolien.

Die Beurteilung des Ausschwitzverhaltens der Deckstrichfolien erlaubt Rückschlüsse auf die Permanenz der verwendeten Weichmacher und anderer Formulierungsbestandteile im ausgelierten System. Eine starke Migration von Formulierungsbestandteilen (welche sich z.B. in der Bildung von Schmierfilmen und/oder Tröpfchen auf der Folienoberfläche äußern kann) hat neben optischen und ästhetischen auch zahlreiche praktische Nachteile. So kommt es durch die erhöhte Klebrigkeit zum Anhaften von Staub und/oder Schmutz, welcher sich nicht oder zumindest nicht vollständig wieder entfernen lässt, und somit in sehr kurzer Zeit zu einem negativen Erscheinungsbild führt. Außerdem wird die Oberflächenhaptik stark beeinträchtigt, es besteht zusätzlich erhöhte Rutschgefahr. Weiterhin kann es durch Wechselwirkungen mit Befestigungsklebstoffen zu einem unkontrollierten Ablösen des Fußbodenbelages kommen. Zur Beurteilung des Ausschwitzverhaltens wird das in Tabelle 7 abgebildete Benotungssystem verwendet. Da es sich beim Ausschwitzen in der Regel um ein sog. "K.O."-Kriterium handelt, ist bei der Bewertung nur eine geringe Abstufung sinnvoll. Die Folien werden im Zeitraum zwischen den Bewertungen bei 25 °C gelagert.

**Tabelle 7: Bewertungssystem für die Bewertung des Ausschwitzverhaltens bei Deckstrichfolien.**

| **Bewertung** | **Bedeutung** |
|---|---|
| 1 | **Sehr gut** (keinerlei Diffusion bzw. Migration erkennbar; keinerlei Filmbildung an der Oberfläche). |
| 3 | **Gut - Befriedigend** (keine offensichtliche Diffusion bzw. Migration erkennbar; minimale Filmbildung an der Oberfläche). |
| 5 | **Mangelhaft** (eindeutige Migrationserscheinungen; "fettige" Haptik; Tröpfchenbildung; Trübung durch Ausscheidung). |

Die Ergebnisse der Oberflächen- und Rückseitenbeurteilung sind in Tabelle 8 zusammengestellt.

**Tabelle 8: Ergebnisse der Oberflächen- und Rückseitenbeurteilung der ausgelierten Deckstrichfolien aus Beispiel 8.**

| **Plastisolrezeptur (gemäß Bsp. 4)** | **1**** | **2**** | **3**** | **4*** |
|---|---|---|---|---|
| Opazität [-] | 9,7 | 8,8 | 8,9 | 9 |
| Gelbwert [-] | 8,4 | 7,4 | 7,3 | 7,7 |
| Beurteilung Ausschwitzverhalten nach 24 h | 1 | 1 | 1 | 1 |
| Beurteilung Ausschwitzverhalten nach 4 Wochen | 1 | 3 | 3 | 3 |

| | | | | |
|---|---|---|---|---|
| ** = Vergleichsbeispiel * = Erfindungsgemäß | | | | |

Bezüglich Opazität und Gelbwert weist die auf Basis des erfindungsgemäßen Plastisols hergestellte Folie einen deutlichen Vorteil gegenüber dem DINP-Standard (1) auf. Demgegenüber ist die Verträglichkeit (Ausschwitzverhalten) leicht schlechter als beim DINP. Das leichte Defizit in der Verträglichkeit gegenüber den DINP-Standardplastisolen kann bei Bedarf vom Fachmann einfach entweder wiederum durch die Zugabe von anderen Weichmachern, insbesondere von schnell gelierenden Weichmachern (z.B. Terephthalate mit C₄ oder C₅-Esterketten bzw. Benzoate mit C₅ bis C₁₃-Estergruppen) oder aber durch eine Anpassung der Verarbeitungsbedingungen (z.B. höhere Geliertemperatur) ausgeglichen werden.

### Beispiel 9:

### Bestimmung der Thermostabilität der Deckstrichfolien

Die Thermostabilitätsuntersuchung erfolgte unter Verwendung von einer speziell für diese Fragestellung entwickelten Rahmenkonstruktion an / in einem Mathis Thermotester (Typ LTE-TS; Fa. Mathis AG). Dabei wird der Prüfrahmen zunächst vollständig in den Ofenbereich eingefahren, und dann mit einem bestimmten Vorschub so aus dem Ofen ausgefahren, dass sich auf dem Probenstreifen Bereiche mit unterschiedlichen Verweilzeiten (= unterschiedlicher Temperaturbelastung) ergeben. Als Grundlage zur Herstellung der Prüfstreifen wurde die in Beispiel 8 hergestellte Folie verwendet. Die Proben wurden zunächst mit einer Schlagschere zugeschnitten. Dabei wurden zuerst die seitlichen Ränder der Folie so entfernt, dass die Folie eine Breite von 20 cm aufwies. Dann wurden je zwei Streifen (20 * 2 cm) abgeschnitten. Die Streifen wurden hintereinander in einer Schiene des erwähnten Rahmens zur Bestimmung der Thermostabilität gelegt, und mit einer Metallklammer und einem Klebeband befestigt, so dass sich eine Gesamtlänge von ca. 40 cm ergab. Die 4 äußersten Schienen im Rahmen wurden nicht belegt (Ausschluß inhomogener Temperaturverteilung im Ofen-Randbereich). Am Mathis Thermotester (Typ LTE-TS Fa. Mathis AG) wurden folgende Parameter eingestellt:
Temperatur: 200 °C
Intervallvorschub des Prüfrahmens: 28 mm
Intervallzeit: 1 min
Lüfterumdrehungszahl: 1800 U/min

Nach eingeregelter Temperatur wurde der Rahmen in der Führung des Thermotesters eingerastet und die Messung gestartet.
Mit Hilfe eines Byk Farbmessgerätes (Spectro Guide 45/0 Fa. Byk Gardner) wurden die L* a* b* incl. eines Gelbwertes Y nach Index D1925 ermittelt. Die eingestellte Lichtart C/2° und die Verwendung eines Probenbeobachters wurden benutzt um optimale Messergebnisse zu erzielen. Die Thermostabilitätsstreifen wurden nun bei jedem Vorschub (28mm) vermessen. Die Messwerte wurden hinter einer weißen Kachel bestimmt. Die schon unmittelbar nach der Herstellung der Folien vorliegende Gelbfärbung (siehe Tabelle 8) wurde als "Nullwert" verwendet, d.h. von den ermittelten Werten abgezogen.

**Tabelle 9: Gelbwerte der in Beispiel 8 hergestellten Deckstrichfolien nach unterschiedlich langer thermischer Belastung bei 200 °C.**

| **Plastisolrezeptur (gemäß Bsp. 4)** | **1**** | **2**** | **3**** | **4*** |
|---|---|---|---|---|
| Gelbwert [-] nach 2 min. @ 200 °C | 0 | 0 | 0 | 0 |
| Gelbwert [-] nach 4 min. @ 200 °C | 2,7 | 1,2 | 0,1 | 0,6 |
| Gelbwert [-] nach 6 min. @ 200 °C | 17,1 | 29 | 5,8 | 7,4 |
| Gelbwert [-] nach 8 min. @ 200 °C | 59,4 | 86 | 26 | 28 |

| | | | | |
|---|---|---|---|---|
| ** = Vergleichsbeispiel * = Erfindungsgemäß | | | | |

Die Folien welche auf Basis des erfindungsgemäßen Plastisols hergestellt wurde, weist überraschender Weise eine im Vergleich zur DINP-Standardprobe (1) aber auch zur Di(isononyl)adipat-Probe (2) eine deutlich verbesserte Thermostabilität auf. Es werden somit transparente PVC-Folien zur Verfügung gestellt, die eine deutlich höhere Toleranz gegenüber Temperaturschwankungen und einer Verlängerung der Ofenverweilzeit aufweisen, als das vom Stand der Technik her bekannt ist.

### Beispiel 10:

### Verwendung von Bernsteinsäurediisononylester (Diisononylsuccinate) in PVC-Deckstrichformulierung (Plastisol) zusammen mit anderen Weichmachern - Herstellung der Deckstrich-Plastisole.

Wie in den Beispielen 4 bis 9 diskutiert, ist eine Anpassung bestimmter Materialparameter der erfindungsgemäßen Formulierungen, Rezepturen bzw. der aus diesen herstellbaren Halbzeuge prinzipiell durch die Kombination der erfindungsgemäßen Bernsteinsäureester mit weiteren Weichmachern möglich. Die folgenden Beispiele sollen die Vorteile dieser Weichmacherkombinationen näher erläutern. Die Herstellung der Plastisole erfolgte gemäß Beispiel 3.1 jedoch mit veränderter Rezeptur. Die verwendeten Einwaagen der Komponenten für die verschiedenen Plastisole sind der nachfolgenden Tabelle (10) zu entnehmen.

**Tabelle 10: PVC-Deckstrich-Rezepturen mit Weichmacherkombinationen. [Alle Angaben in phr (= Massenteile pro 100 Massenteile PVC)]**

| **Plastisolrezeptur** | **1**** | **2*** | **3*** | **4*** | **5*** | **6*** | **7*** | **8*** |
|---|---|---|---|---|---|---|---|---|
| Vestolit B 7021 --Ultra | 100 | 100 | 100 | 100 | 100 | 100 | 100 | 100 |
| VESTINOL® 9 | 50 | | | | | | | |
| Di(nonyl)succinat gemäß Bsp. 2.3 | | 50 | 45 | 40 | 30 | 20 | 20 | 20 |
| Unimoll AGF | | | | | 10 | 20 | | |
| Grindstedt's Soft'n Safe | | | | | | | 20 | |
| Isosorbiddisiononylester | | | | | | | | 20 |
| Drapex 39 | 3 | 3 | 3 | 3 | 3 | 3 | 3 | 3 |
| Mark CZ 149 | 2 | 2 | 2 | 2 | 2 | 2 | 2 | 2 |

| | | | | | | | | |
|---|---|---|---|---|---|---|---|---|
| ** = Vergleichsbeispiel * = Erfindungsgemäß | | | | | | | | |

Die verwendeten Stoffe und Substanzen, welche sich nicht bereits aus den vorangegangenen Beispielen ergeben, werden im Folgenden näher erläutert:
**Unimoll AGF:** Glycerinacetatgemisch; Weichmacher; Lanxess AG.
**Grindstedt's Soft'n Safe:** Octadecansäure-12-(acetyloxy)-2,3-bis(acetyloxy)propylester; Glycerintriester hergestellt auf Basis von Ricinusöl; Weichmacher; Fa. Danisco A/S.
**Isosorbiddiisononylester:** Laborprodukt hergestellt gemäß DE 102007006442A1, Beispiel 2.

### Beispiel 11 :

### Bestimmung der Plastisolviskosität von Deckstrichplastisolen enthaltend Dinonylsuccinate und weitere Weichmacher nach einer Lagerdauer von 24 h (bei 25 °C).

Die Messung der Viskositäten der in Beispiel 10 hergestellten Plastisole erfolgte mit einem Rheometer Physica MCR 101 (Fa. Paar-Physica), gemäß der unter Analytik, Punkt 11 beschriebenen Vorgehensweise. Die Ergebnisse werden in der nachfolgenden Tabelle (11) exemplarisch für die Schergeschwindigkeiten 100/s, 10/s, 1/s und 0,1/s dargestellt.

**Tabelle 11: Scherviskosität der Plastisole aus Beispiel 10 nach 24 h Lagerung bei 25 °C.**

| **Plastisolrezeptur gemäß Bsp. 10** | **1**** | **2*** | **3*** | **4*** | **5*** | **6*** | **7*** | **8*** |
|---|---|---|---|---|---|---|---|---|
| Scherviskosität bei Schergeschwindigkeit= 100/s [Pa*s] | 6,2 | 0,95 | 1,5 | 2,7 | 3,5 | 5,5 | 12,2 | 12,7 |
| Scherviskosität bei Schergeschwindigkeit= 10/s [Pa*s] | 2,9 | 0,58 | 0,77 | 1,1 | 1,3 | 1,9 | 3,3 | 2,8 |
| Scherviskosität bei Schergeschwindigkeit= 1/s [Pa*s] | 2,7 | 0,62 | 0,82 | 1,2 | 1,5 | 2,3 | 3,2 | 2,3 |
| Scherviskosität bei Schergeschwindigkeit= 0,1/s [Pa*s] | 3,3 | 0,87 | 1,2 | 1,8 | 2,5 | 4,4 | 5,1 | 3,3 |

| | | | | | | | | |
|---|---|---|---|---|---|---|---|---|
| ** = Vergleichsbeispiel * = Erfindungsgemäß | | | | | | | | |

Die erfindungsgemäßen Plastisole welche nur die erfindungsgemäßen Diisononylsuccinate enthalten ((2) bis (4)) liegen in ihrer Plastisolviskosität alle weit unterhalb der vom DINP-Standardplastisol (1) erreichten Werte, und zwar auch noch bei einer signifikant (um ca. 20%) reduzierten Weichmachermenge. Dies bedeutet insbesondere, dass diese erfindungsgemäßen Plastisole in vielen technisch relevanten Auftragsverfahren (z.B. dem Rakelauftrag) schneller appliziert werden können. Auch in Kombination aus erfindungsgemäßem Diisononylsuccinat und anderen Weichmachern ((5) bis (8) werden zum großen Teil (weiterhin mit um 20% reduzierter Gesamtweichmachermenge) deutlich geringere Plastisolviskositäten erhalten als beim DINP-Standardplastisol. Lediglich die Mischung aus erfindungsgemäßem Diisononylsuccinat und dem Glycerinester Grindsted's Soft'n Safe (7) zeigt eine gegenüber dem DINP-Standardplastisol (leicht) erhöhte Plastisolviskosität. Es werden somit erfindungsgemäße Plastisole zur Verfügung gestellt, die eine gegenüber dem bekannten DINP-Standard signifikant verbesserte Verarbeitbarkeit aufweisen, und insbesondere eine deutlich höhere Verarbeitungsgeschwindigkeit in vielen relevanten Auftragsverfahren erlauben.

### Beispiel 12:

### Bestimmung des Gelierverhaltens der in Beispiel 10 hergestellten PVC-Deckstrich-Plastisole.

Die Untersuchung des Gelierverhaltens der in Beispiel 10 hergestellten PVC-Deckstrich-Plastisole erfolgte wie unter Analytik, Punkt 12. (siehe oben) beschrieben, mit einem Physica MCR 101 im Oszillationsmodus nach einer Lagerung der Plastisole bei 25 °C für 24 h. Die Ergebnisse werden in der nachfolgenden Tabelle (12) dargestellt.

**Tabelle 12: Aus den Gelierkurven (Viskositätskurven) bestimmte Eckpunkte des Gelierverhaltens der gemäß Beispiel 10 hergestellten PVC-Deckstrich-Plastisole.**

| **Plastisolrezeptur (gemäß Bsp. 10)** | **1**** | **2*** | **3*** | **4*** | **5*** | **6*** | **7*** | **8*** |
|---|---|---|---|---|---|---|---|---|
| Erreichen einer Plastisolviskosität von 1.000 Pa*s bei [°C] | 87 | 120 | 111 | 94 | 85 | 82 | 83 | 86 |
| Erreichen einer Plastisolviskosität von 10.000 Pa*s bei [°C] | 99 | 136 | 133 | 127 | 124 | 94 | 90 | 109 |
| Maximale Plastisolviskosität [Pa*s] | 26700 | 14700 | 16300 | 15900 | 17300 | 29700 | 27900 | 27100 |
| Temperatur beim Erreichen der maximalen Plastisolviskosität [°C] | 139 | 144 | 139 | 135 | 144 | 139 | 142 | 144 |

| | | | | | | | | |
|---|---|---|---|---|---|---|---|---|
| ** = Vergleichsbeispiel * = Erfindungsgemäß | | | | | | | | |

Die erfindungsgemäßen Diisononylsuccinate alleine bewirken erst bei deutlich geringerer Weichmacherkonzentration im Plastisol (-20% im Vergleich zum DINP-Standardplastisol (1)) eine ähnliche Gelierung wie DINP. Dabei wird zwar eine ähnliche Anfangsgeschwindigkeit erreicht (4), der weitere Verlauf der Gelierung, insbesondere aber die maximal erreichbare Plastisolviskosität im ausgelierten Zustand, bleibt aber deutlich hinter der des DINP-Standardplastisols zurück. Die Kombination aus erfindungsgemäßen Isononylsuccinaten mit anderen Weichmachern kann diesen Unterschied allerdings ohne weiteres ausgleichen und führt in einigen Fällen ((6) und (7)) sogar zu einer schnelleren Gelierung und dem Erreichen höherer Maximalviskositäten im ausgelierten Zustand. Insbesondere die Kombination aus erfindungsgemäßen Succinatweichmachern und weiteren Weichmachern, welche auf Glycerinestern beruhen, erscheint somit besonders vorteilhaft zu sein, zumal im Vergleich mit dem DINP-Standardplastisol eine erhebliche Verringerung der Gesamtweichmacherkonzentration im Plastisol (und damit eine deutlich höhere Wirtschaftlichkeit) erzielt werden kann, wobei gleichzeitig keine Orthophthalate anwesend sind, und ein außerordentlich hoher Anteil der Weichmacher auf Basis regenerativer Rohstoffe hergestellt werden kann.

### Beispiel 13:

### Bestimmung der Wasseraufnahme und des Auswaschverhaltens an Deckstrich-Prüfkörpern

Wasseraufnahme und Auswaschverhalten sind zwei wesentliche Kriterien bei der Beurteilung der Güte von PVC-Fußbodenbelägen. Nimmt ein PVC-Fußboden in größerem Umfang Wasser auf, so verändern sich dadurch einerseits seine Materialeigenschaften, andererseits auch sein optisches Aussehen (z.B. Eintrübung). Eine hohe Wasseraufnahme ist demnach in der Regel unerwünscht. Das Auswaschverhalten ist ein zusätzliches Kriterium für die Permanenz der Formulierungsbestandteile unter Gebrauchsbedingungen (z.B. bei Fußbodenbelägen oder Dachbahnen). Dies gilt insbesondere für Stabilisatoren, Weichmacher und/oder ihre Bestandteile, da eine Konzentrationsminderung im PVC-Fußboden bei diesen Rezepturbestandteilen sowohl die Materialeigenschaften verschlechtern als auch die Lebensdauer des Fußbodenbelages dramatisch reduzieren kann. Insbesondere in den oberen Fußbodenschichten -wie zum Beispiel im Transparent-Deckstrich- sind daher Wasseraufnahme und Auswaschverhalten von besonderer Bedeutung.

Für die Bestimmung der Wasserbeständigkeit wurden ausgelierte 1 mm-Polymerfilme (Gelierbedingungen im Mathisofen: 200 °C/2 min.) verwendet. Als Prüfkörper wurden aus den Folien Kreise mit 3 cm Durchmesser ausgeschnitten. Vor der Wasserlagerung wurden die Kreise für 24 Stunden in einem mit Trockenmittel (KC-Trockenperlen) ausgerüsteten Exsikkator bei 25 °C gelagert. Das Ausgangsgewicht (Einwage) wurde mit einer Analysenwaage auf 0,1 mg genau bestimmt. Die Kreise wurden nun in einem mit VE-Wasser gefülltem Schüttelbad (Typ: WNB (40 I); Hersteller: Fa. Memmert) bei einer Temperatur von 30 °C für 24 Stunden mit geeigneten Probenhaltern unter der Wasseroberfläche gelagert und kontinuierlich bewegt. Nach der Lagerung wurden die Kreise dem Wasserbad entnommen, abgetrocknet und ausgewogen (Gewicht nach 24h). Die ausgewogenen Kreise wurden erneut im Wasserbad platziert und nach 7 Tagen erneut im abgetrockneten Zustand ausgewogen (Gewicht nach 7 Tagen). Nach der zweiten Auswage wurden die Kreise wiederum für 24 Stunden in einem mit Trockenmittel (KC-Trockenperlen) ausgerüsteten Exsikkator bei 25 °C gelagert und anschließend nochmals ausgewogen (Endauswaage = Gewicht nach Trocknung). Die Gewichtsänderungen wurden prozentual errechnet und sind in Tabelle 13 dargestellt.

**Tabelle 13: Wasseraufnahme und Auswaschverhaltens bestimmt an Deckstrich-Prüfkörpern hergestellt gemäß Beispiel 13.**

| Plastisolrezeptur gemäß Bsp. 10 | **1**** | **2*** | **3*** | **4*** | **5*** | **6*** | **7*** | **8*** |
|---|---|---|---|---|---|---|---|---|
| Gewichtsänderung nach 1 Tag [ma%] | + 0,9 | + 1,2 | + 1,1 | + 1,1 | + 0,9 | + 0,8 | + 1,1 | + 0,9 |
| Gewichtsänderung nach 7 Tagen [ma%] | + 1,2 | + 1,6 | + 1,5 | + 1,5 | + 1,3 | +1,1 | + 1,4 | +1,2 |
| Gewichtsänderung nach Trockung [ma%] | + 0,2 | + 0,2 | + 0,1 | + 0,2 | -0,1 | -0,3 | + 0,1 | ± 0 |

| | | | | | | | | |
|---|---|---|---|---|---|---|---|---|
| ** = Vergleichsbeispiel * = Erfindungsgemäß | | | | | | | | |

Die Prüfkörper, welche die erfindungsgemäßen Diisononylsuccinate enthalten, weisen im Wesentlichen eine dem DINP-Standard ähnliche Wasseraufnahme bzw. ein ähnliches Auswaschverhalten auf, wobei in beiden Fällen nur geringfügige Effekte auftreten. Die Kombination aus erfindungsgemäßen Diisononylsuccinaten und Isosorbidester weist eine besonders geringe Wasseraufnahme auf, und zeigt gleichzeitig weder eine Wasserbindung an das Substrat noch eine Auswaschung, was als besonders vorteilhaft zu werten ist.

### Beispiel 14 :

### Bestimmung der weichmachenden Wirkung bzw. der Weichmachereffizienz an Gießlingen durch Bestimmung der Shore-Härte (Shore A)

Die Herstellung der Gießlinge erfolgte gemäß der in Beispiel 7 beschriebenen Vorgehensweise, jedoch unter Verwendung der gemäß Beispiel 10 hergestellten Plastisole. Die Bestimmung der Shore-Härte erfolgte gemäß der unter Analytik, Punkt 14 (siehe oben) beschriebenen Vorgehensweise. Die Ergebnisse der Härtebestimmung sind in der nachfolgenden Tabelle (14) zusammengestellt.

**Tabelle 14: Härte nach Shore A an Gießlingen hergestellt aus Diisononylsuccinate und weitere Weichmacher enthaltenden Deckstrichplastisolen (gemäß Beispiel 10).**

| Plastisolrezeptur gemäß Bsp. 10 | **1**** | **2*** | **3*** | **4*** | **5*** | **6*** | **7*** | **8*** |
|---|---|---|---|---|---|---|---|---|
| Shore A | 81 | 79 | 83 | 87 | 86 | 85 | 86 | 89 |

| | | | | | | | | |
|---|---|---|---|---|---|---|---|---|
| ** = Vergleichsbeispiel * = Erfindungsgemäß | | | | | | | | |

Die erfindungsgemäßen Diisononylsuccinate weisen bei alleiniger Verwendung ((2) bis (4)) eine ähnlich weichmachende Wirkung wie DINP auf, selbst bei einer Weichmacherreduktion um 10% (3). Bei Reduktion der Weichmachergesamtmenge um 20% sinkt die weichmachende Wirkung leicht ab und kann auch durch die Verwendung von Weichmacherkombinationen mit Glycerinestern und Isosorbidestern nicht ohne weiters auf das DINP-Niveau angehoben werden. Als eine Möglichkeit dieses Problem zu lösen bietet sich dem Fachmann der Einsatz von Weichmachern mit schneller Gelierung (sog. Schnellgelierer) an, z.B. von Benzoaten, Citronensäureestern, Alkylsulfonaten usw.

### Beispiel 15:

### Bestimmung von Opazität, Gelbwert und Ausschwitzverhalten der Deckstrichfolien.

Die Herstellung der Deckstrichfolien erfolgte wie in Beispiel 3.1 beschrieben, jedoch unter Verwendung der gemäß Beispiel 10 hergestellten Plastisole.
Die Bestimmung der Opazität erfolgte gemäß der unter Analytik, Punkt 15 (siehe oben) beschriebenen Vorgehensweise.
Die Bestimmung der Gelbwerte der Deckstrichfolien, erfolgte gemäß der unter Analytik, Punkt 13 (siehe oben) beschriebenen Vorgehensweise.
Die Beurteilung des Ausschwitzverhaltens erfolgte gemäß der in Beispiel 8 beschriebenen Verfahrensweise unter Verwendung des in Tabelle 7 dargestellten Benotungssystems.

Die Ergebnisse der Oberflächen- und Rückseitenbeurteilung sind in Tabelle 15 zusammengestellt.

**Tabelle 15: Ergebnisse der Oberflächen- und Rückseitenbeurteilung der ausgelierten Deckstrichfolien aus Beispiel 15.**

| **Plastisolrezeptur (gemäß Bsp. 10)** | **1**** | **2*** | **3*** | **4*** | **5*** | **6*** | **7*** | **8*** |
|---|---|---|---|---|---|---|---|---|
| Opazität [-] | 10,1 | 10 | 10,7 | 12,4 | 12,9 | 13,5 | 11,5 | 13,7 |
| Gelbwert [-] | 8,5 | 8,3 | 9 | 9,7 | 10,1 | 10,3 | 9,2 | 10,3 |
| Beurteilung Ausschwitzverhalten nach 24 h (bei 25 °C) | 1 | 1 | 1 | 1 | 1 | 1 | 1 | 1 |
| Beurteilung Ausschwitzverhalten nach 4 Wochen (bei 25 °c) | 1 | 3 | 3 | 3 | 5 | 5 | 3 | 3 |

| | | | | | | | | |
|---|---|---|---|---|---|---|---|---|
| ** = Vergleichsbeispiel * = Erfindungsgemäß | | | | | | | | |

Verwendete man allein die erfindungsgemäßen Diisononylsuccinate ((2) bis (4)), so werden sowohl bezüglich der Opazität/Transparenz als auch bezüglich des Gelbwertes ähnliche Werte wie beim DINP-Standard (1) erzielt. Bei Kombinationen aus erfindungsgemäßen Diisononylsuccinaten und weiteren Weichmachern kommt es zu einer Verringerung der Transparenz bei gleichzeitigem Ansteigen des Gelbwertes. Die Proben liegen dabei aber noch immer im akzeptablen Bereich, zumal die Gesamtweichmachermenge im Vergleich mit der DINP-Probe erheblich reduziert ist. Bezüglich des Ausschwitzverhaltens weisen alle Proben mit Ausnahme der Kombination aus erfindungsgemäßen Diisononylsuccinaten und Unimoll AGF eine akzeptable Performance auf. Die leichten Nachteile sowohl bei der Opazität als auch beim Ausschwitzverhalten können vom Fachmann leicht durch verarbeitungs- und/oder formulierungstechnischen Maßnahmen, wie beispielsweise durch Zugabe von geringen Mengen an Weichmachern mit (im Vergleich zu den verwendeten erfindungsgemäßen Diisonoylsuccinaten) schnellerer Gelierung kompensiert werden.

### Beispiel 16:

### Herstellung von gefüllten und pigmentierten expandierbaren / schäumbaren PVC-Plastisolen zur Verwendung für Effektschäume.

Im Folgenden sollen die Vorteile der erfindungsgemäßen Plastisole an Hand von Füllstoff und Pigment enthaltenden thermisch expandierbaren PVC-Plastisolen verdeutlicht werden, die sich zur Herstellung von Effektschäumen (Schäumen mit besonderer Oberflächenstruktur) eignen. Diese Schäume werden nach dem aus dem Textilbereich bekannten Erscheinungsmuster häufig auch als "Boucle-Schäume" bezeichnet. Die nachstehenden erfindungsgemäßen Plastisole stehen dabei u.a. beispielhaft für thermisch expandierbare Plastisole die bei der Herstellung von Wandbelägen zum Einsatz kommen. Insbesondere sind die nachstehenden erfindungsgemäßen Plastisole beispielhaft für Schaumschichten welche in PVC-Tapeten Verwendung finden.

Die Herstellung der Plastisole erfolgte analog Beispiel 3.5 jedoch mit veränderter Rezeptur. Die verwendeten Einwaagen der Komponenten für die verschiedenen Plastisole sind der nachfolgenden Tabelle (16) zu entnehmen.

**Tabelle 16: Zusammensetzung der gefüllten und pigmentierten expandierbaren PVC-Plastisole aus Beispiel 10 [Alle Angaben in phr (= Massenteile pro 100 Massenteile PVC)].**

| **Plastisolrezeptur** | **1**** | **2*** | **3*** | **4*** | **5*** | **6*** | **7*** | **8*** |
|---|---|---|---|---|---|---|---|---|
| Vestolit E 7012 S | 100 | 100 | 100 | 100 | 100 | 100 | 100 | 100 |
| VESTINOL® 9 | 54 | | | | | | | |
| Di(nonyl)succinat gemäß Bsp. 2.3 | | 54 | 49 | 44 | 34 | 24 | 24 | 24 |
| Unimoll AGF | | | | | 10 | 20 | | |
| Grindstedt's Soft'n Safe | | | | | | | 20 | |
| Isosorbiddisiononylester | | | | | | | | 20 |
| Unicell D200A | 5 | 5 | 5 | 5 | 5 | 5 | 5 | 5 |
| Calibrite-OG | 30 | 30 | 30 | 30 | 30 | 30 | 30 | 30 |
| Kronos 2220 | 8 | 8 | 8 | 8 | 8 | 8 | 8 | 8 |
| Baerostab KK 48-1 | 3 | 3 | 3 | 3 | 3 | 3 | 3 | 3 |
| Isopar J | 3 | 3 | 3 | 3 | 3 | 3 | 3 | 3 |
| Isopropanol | 3 | 3 | 3 | 3 | 3 | 3 | 3 | 3 |

| | | | | | | | | |
|---|---|---|---|---|---|---|---|---|
| ** = Vergleichsbeispiel * = Erfindungsgemäß | | | | | | | | |

Die verwendeten Stoffe und Substanzen, welche sich nicht bereits aus den vorangegangenen Beispielen ergeben, werden im Folgenden näher erläutert:
**Vestolit E 7012 S:** Emulsions-PVC (Homopolymer) mit einem K-Wert (bestimmt gemäß DIN EN ISO 1628-2) von 67; Fa. Vestolit GmbH.
**Unicell D200A:** Azodicarbonamid; thermisch aktivierbares Treibmittel; Fa. Tramaco GmbH.
**Calibrite-OG:** Calciumcarbonat; Füllstoff; Fa. OMYA AG.
**KRONOS 2220:** Mit Al und Si stabilisiertes Rutilpigment (TiO₂); Weißpigment; Fa. Kronos Worldwide Inc.
**Baerostab KK 48-1:** Kalium/Zink-"Kicker"; Zersetzungskatalysator für thermische Treibmittel; setzt die substanzeigene Zersetzungstemperatur der Treibmittels herab; gleichzeitig auch stabilisierende Wirkung; Fa. Baerlocher GmbH.
**Isopar J:** Isoparaffin, Colösungsmittel zur Absenkung der Plastisolviskosität; Fa. Möller Chemie.
**Isopropanol:** Colösungsmittel zur Absenkung der Plastisolviskosität sowie Additiv zur Verbesserung der Schaumstruktur (Fa. Brenntag AG).

### Beispiel 17:

### Bestimmung der Plastisolviskosität der gefüllten und pigmentierten thermisch expandierbaren Plastisole aus Beispiel 16 nach einer Lagerdauer von 24 h (bei 25°C).

Die Messung der Viskosität der in Beispiel 16 hergestellten Plastisole erfolgte wie unter Analytik, Punkt 11. (siehe oben) beschrieben, mit einem Rheometer Physica MCR 101 (Fa. Paar-Physica). Die Ergebnisse werden in der nachfolgenden Tabelle (17) exemplarisch für die Schergeschwindigkeiten 100/s, 10/s, 1/s und 0,1/s dargestellt.

**Tabelle 17: Scherviskosität der Plastisole aus Beispiel 16 nach 24 h Lagerung bei 25 °C.**

| **Plastisolrezeptur gemäß Bsp. 16** | **1**** | **2*** | **3*** | **4*** | **5*** | **6*** | **7*** | **8*** |
|---|---|---|---|---|---|---|---|---|
| Scherviskosität bei Schergeschwindigkeit= 100/s [Pa*s] | 4 | 1,1 | 1,4 | 1,9 | 2,1 | 2,5 | 3,8 | 3,3 |
| Scherviskosität bei Schergeschwindigkeit= 10/s [Pa*s] | 4,9 | 1,5 | 2 | 2,6 | 3 | 3,4 | 4,7 | 3,9 |
| Scherviskosität bei Schergeschwindigkeit= 1/s [Pa*s] | 9,1 | 3,5 | 4,6 | 6,2 | 7,1 | 8,1 | 10,5 | 8,5 |
| Scherviskosität bei Schergeschwindigkeit= 0,1/s [Pa*s] | 22,5 | 10,8 | 14,4 | 19,7 | 23 | 26,8 | 32,8 | 25,9 |

| | | | | | | | | |
|---|---|---|---|---|---|---|---|---|
| ** = Vergleichsbeispiel * = Erfindungsgemäß | | | | | | | | |

Alle Plastisole welche die erfindungsgemäßen Diisononylsuccinate enthalten weisen ab einer Schergeschwindigkeit von 10* s⁻¹ eine deutlich niedrigere Scherviskosität als das DINP-Plastisol (= Standard) auf. Dabei ist sowohl der Einsatz des reinen Diisononylsuccinates als auch der Einsatz von Weichmacherkombinationen vorteilhaft. Insbesondere ist zu beachten, dass selbst eine um ca. 20% verringerte Weichmachermenge ((4) im Vergleich zu (1)) noch zu einer deutlich niedrigeren Plastisolviskosität führt. Es ist somit anzunehmen, dass im Vergleich mit DINP eine deutlich geringere Weichmachermenge eingesetzt werden kann. Es werden somit erfindungsgemäße Plastisole zur Verfügung gestellt, die im Vergleich mit dem derzeitigen Standard (DINP) eine deutlich verbesserte Verarbeitbarkeit aufweisen, und insbesondere deutlich schnellere Verarbeitungsgeschwindigkeiten zulassen, wobei gleichzeitig eine deutliche Reduktion der Weichmachermenge möglich ist.

### Beispiel 18:

### Bestimmung des Gelierverhaltens der gefüllten und pigmentierten thermisch expandierbaren Plastisole aus Beispiel 16

Die Untersuchung des Gelierverhaltens der in Beispiel 16 hergestellten gefüllten und pigmentierten thermisch expandierbaren Plastisole, erfolgte wie unter Analytik, Punkt 12. (siehe oben) beschrieben, mit einem Physica MCR 101 im Oszillationsmodus nach einer Lagerung der Plastisole bei 25 °C für 24 h. Die Ergebnisse werden in der nachfolgenden Tabelle (18) dargestellt.

**Tabelle 18: Aus den Gelierkurven (Viskositätskurven) bestimmte Eckpunkte des Gelierverhaltens der gemäß Beispiel 16 hergestellten gefüllten und pigmentierten expandierbaren Plastisole.**

| **Plastisolrezeptur (gemäß Bsp. 16)** | **1**** | **2*** | **3*** | **4*** | **5*** | **6*** | **7*** | **8*** |
|---|---|---|---|---|---|---|---|---|
| Erreichen einer Plastisolviskosität von 1.000 Pa*s bei [°C] | 77 | 106 | 97 | 87 | 82 | 77 | 78 | 81 |
| Erreichen einer Plastisolviskosität von 10.000 Pa*s bei [°C] | 103 | - | - | - | 130 | 120 | 107 | 112 |
| Maximale Plastisolviskosität [Pa*s] | 13800 | 4100 | 6000 | 8500 | 10700 | 10900 | 11600 | 11200 |
| Temperatur beim Erreichen der maximalen Plastisolviskosität [°C] | 117 | 142 | 140 | 137 | 132 | 124 | 126 | 126 |

| | | | | | | | | |
|---|---|---|---|---|---|---|---|---|
| ** = Vergleichsbeispiel * = Erfindungsgemäß | | | | | | | | |

Bei gleichem Weichmachergehalt (Vergleich (1) gegen (2)) liegt die Geliergeschwindigkeit der erfindungsgemäßen Succinat-Weichmacher deutlich unterhalb der Geschwindigkeit des DINP-Standards bzw. die Geliertemperatur der erfindungsgemäßen Succinat-Weichmacher deutlich oberhalb der Geliertemperatur des DINP-Standards. Die Weichmacher-Konzentrationsreihe ((2) bis (4)) zeigt aber deutlich, dass die Geliergeschwindigkeit bzw. Die Geliertemperatur durch eine Reduktion des Weichmachergehaltes wie ihn schon die Plastisolviskosität (siehe Beispiel 17) nahe legt, deutlich in Richtung des DINP-Standardes hin verschoben werden kann. Auch die maximal erreichbare Plastisolviskosität im aufgeschäumten und ausgelierten Zustand bewegt sich deutlich in Richtung DINP-Plastisol. Durch eine Kombination mit weiteren Weichmachern ((5) bis (8)) lassen sich ohne Probleme die vom DINP-Standrad erreichten Werte nachstellen, wobei immer noch eine Reduktion der Gesamtweichmachermenge um ca. 20% möglich bleibt. Es werden somit erfindungsgemäße Plastisole zur Verfügung gestellt, die bei deutlich verringerter Weichmachermenge die vom DINP-Standard bekannten guten Geliereigenschaften zeigen, und gleichzeitig frei von ortho-Phthalaten sind bzw. sein können.

### Beispiel 19:

### Herstellung und Beurteilung des Effektschaumes aus gefüllten und pigmentierten thermisch expandierbaren Plastisolen aus Beispiel 16.

Die Herstellung der Effektschaum-Tapeten wurde unter Verwendung der gemäß Beispiel 16 hergestellten Plastisole gemäß der in Beispiel 3.5 beschriebenen Vorgehensweise durchgeführt, wobei die Plastisole vor der Verarbeitung für 2h bei 25 °C gelagert wurden. Die Ermittlung der Gelbwerte erfolgte an den ausgelierten Proben wie unter Analytik, Punkt 13 beschrieben (siehe oben).

Bei der Beurteilung des Expansionsverhaltens wird die DINP-Probe als Vergleichsstandard herangezogen. Ein normales Expansionsverhalten (= "O.K.") entspricht also dem Verhalten der DINP-Probe.

Bei der Beurteilung der Oberflächengüte bzw. der Oberflächenstruktur wird vor allem die Gleich- bzw. Regelmäßigkeit der Oberflächenstrukturen bewertet. Auch die dimensionale Ausdehnung der einzelnen Effektbestandteile wird in die Bewertung mit einbezogen.

Hinzu kommt die *Beurteilung der Rückseite* (Papier) hinsichtlich eines Austretens bzw. einer Migration von Rezepturbestandteilen. Das der Bewertung der Oberflächenstruktur zu Grunde liegende Benotungssystem ist in der nachfolgenden Tabelle (19) abgebildet.

**Tabelle 19: Bewertungssystem für die Beurteilung der Oberflächengüte von Effektschäumen.**

| **Bewertung** | **Bedeutung** |
|---|---|
| 1 | **Sehr gute Oberflächenstruktur** (Sehr hohe Regelmäßigkeit und Gleichförmigkeit der Oberflächeneffekte; Größe der Einzeleffekte passend). |
| 2 | **Gute Oberflächenstruktur** (Hohe Regelmäßigkeit und Gleichförmigkeit der Oberflächeneffekte; Größe der Einzeleffekte passend). |
| 3 | **Befriedigende Oberflächenstruktur** (Regelmäßigkeit und Gleichförmigkeit der Oberflächeneffekte akzeptabel; Größe der Einzeleffekte angemessen). |
| 4 | **Ausreichende Oberflächenstruktur** (leichte Unregelmäßigkeiten oder Ungleichförmigkeiten in der Oberflächenstruktur; Größe der Einzeleffekte leicht unbalanciert). |
| 5 | **Mangelhafte Oberflächenstruktur** (Unregelmäßigkeiten und Ungleichförmigkeiten in der Oberflächenstruktur; Größe der Einzeleffekte unbalanciert). |
| 6 | **Ungenügende Oberflächenstruktur** (stark unregelmäßige und ungleichförmige Oberflächeneffekte; Größe der Einzeleffekte unpassend (viel zu groß / viel zu klein)). |

Das der Beurteilung der Tapeten-Rückseiten (Migration) zu Grunde liegende Benotungssystem ist in der nachfolgenden Tabelle (20) abgebildet.

**Tabelle 20: Bewertungssystem für die Rückseitenbeurteilung von Effektschäumen.**

| **Bewertung** | **Bedeutung** |
|---|---|
| 1 | **Sehr gut** (keinerlei Diffusion bzw. Migration erkennbar; keinerlei abweichende Färbung im Randbereich). |
| 2 | **Gut** (keinerlei Diffusion bzw. Migration erkennbar; minimal abweichende Färbung im Randbereich). |
| 3 | **Befriedigend** (minimale Diffusion bzw. Migration erkennbar; eindeutig abweichende Färbung im Auftragsbereich). |
| 4 | **Ausreichend** (leichte Diffusion bzw. Migration erkennbar; eindeutig abweichende Färbung im Auftragsbereich). |
| 5 | **Mangelhaft** (eindeutige Migrationserscheinungen; leicht "fettige" Haptik; starker Farbunterschied im gesamten Auftragsbereich). |
| 6 | **Ungenügend** (starke Migrationserscheinungen; stark "fettige" Haptik; extremer Farbunterschied im gesamten Auftragsbereich). |

Die Oberflächenstruktur eines Effektschaumes (d.h. eines Schaumes, welcher eine besondere / besonders ausgeprägte Oberflächenstrukturierung aufweisen soll) wird im Wesentlichen durch die Bestandteile und die Verarbeitungseigenschaften des zur Herstellung eingesetzten Plastisols bestimmt. Insbesondere sind hier die Plastisolviskosität, das Verlaufsverhalten des Plastisols (z.B. charakterisiert über den Verlauf der Plastisolviskosität als Funktion der Schergeschwindigkeit), das Gelierverhalten des Plastisols (u.a. ausschlaggebend für die Größe und Verteilung der Gasblasen), der Einfluss der verwendeten Weichmacher auf die Zersetzung des Treibmittels (sog. "Auto-Kick-Effekte"), sowie die Wahl und Kombination von Treibmittel(n) und Zersetzungskatalysator(en) zu nennen. Diese werden wesentlich durch die Wahl der Einsatzstoffe, insbesondere der verwendeten Weichmacher, beeinflusst und lassen sich so gezielt steuern.

Die Beurteilung der Rückseite des beschichteten Papiers erlaubt Rückschlüsse auf die Permanenz der verwendeten Weichmacher und anderer Formulierungsbestandteile im ausgelierten System. Eine starke Migration von Formulierungsbestandteilen hat neben optischen und ästhetischen auch zahlreiche praktische Nachteile. So kommt es durch die erhöhte Klebrigkeit zum Anhaften von Staub, welcher sich nicht oder zumindest nicht vollständig wieder entfernen lässt und somit in sehr kurzer Zeit zu einem negativen Erscheinungsbild führt. Hinzu kommt, dass sich Migration von Formulierungsbestandteilen in der Regel stark negativ auf die Bedruckbarkeit bzw. Beständigkeit einer Bedruckung auswirkt. Weiterhin kann es durch Wechselwirkungen mit Befestigungsklebstoffen (z.B. Tapetenkleber) zu einem unkontrollierten Ablösen eines Wandbelages kommen.

Bei der Beurteilung von thermisch expandierbaren Plastisolen ist der Gelbwert in zweierlei Hinsicht von Interesse. Zum einen zeigt er den Zersetzungsgrad des Treibmittels (= gelb im unzersetzten Zustand) an, zum anderen ist er ein Maß für die Thermostabilität (Verfärbungen in Folge thermischer Belastung).

Die Ergebnisse der Oberflächen- und Rückseitenbeurteilung sind in Tabelle 17 zusammengestellt.

**Tabelle 21: Ergebnisse der Oberflächen- und Rückseitenbeurteilung der ausgelierten Effektschäume aus Beispiel 19.**

| **Plastisolrezeptur (gemäß Bsp. 16)** | **1**** | **2*** | **3*** | **4*** | **5*** | **6*** | **7*** | **8*** |
|---|---|---|---|---|---|---|---|---|
| Expansionsverhalten | - | O.K. | O.K. | O.K. | O.K. | O.K. | O.K. | O.K. |
| Gelbwert | 9,1 | 9,3 | 10,1 | 10,1 | 10,2 | 10,9 | 9 | 10,8 |
| Bewertung Oberflächengüte / -struktur | 1 | 4 | 4 | 4 | 3 | 3 | 2 | 3 |
| Bewertung der Rückseite nach 24 h | 1 | 1 | 1 | 1 | 1 | 1 | 1 | 1 |
| Bewertung der Rückseite nach 168 h | 1 | 2 | 2 | 2 | 2 | 2 | 2 | 2 |

| | | | | | | | | |
|---|---|---|---|---|---|---|---|---|
| ** = Vergleichsbeispiel * = Erfindungsgemäß | | | | | | | | |

Das Expansionsverhalten aller Proben ist mit dem der DINP-Standardprobe (1) vergleichbar. Auch die Gelbwerte der Proben liegen auf ähnlichem Niveau, wobei je nach verwendeter Weichmacherkonzentration im Plastisol und dem verwendeten zusätzlichen Weichmacher eine gewisse Schwankungsbreite auftritt. Die beiden Plastisole, die Unimoll AGF enthalten haben insofern einen gewissen Nachteil, als das Unimoll AGF selbst bereits eine deutlich erkennbare gelbbraune Färbung aufweist. Bei der Oberflächengüte ist noch Potential bezüglich einer Rezepturoptimierung zu erkennen, wobei die Güte hier klar vom Verwendeten Coweichmacher abhängt. In allen Fällen ist keine Migration von Formulierungsbestandteilen ins Tapetenpapier zu erkennen. Insgesamt schneidet die Kombination aus erfindungsgemäßen Isononylsuccinat und bestimmten Glycerinestern (7) am besten ab, und liefert ein dem DINP-Standard vergleichbares Ergebnis. Es werden somit Plastisole zur Verfügung gestellt, die bei deutlich erhöhter Weichmachereffizienz (d.h. deutlich geringerer Weichmachermenge) und stark verbesserter Verarbeitungsfähigkeit die Herstellung von Effektschäumen mit zum derzeitigen Standard DINP vergleichbarer Qualität erlauben, wobei gleichzeitig ein bestimmter Anteil der verwendeten Weichmacher (optional) auf nachwachsenden Rohstoffen basiert und (optional) auch eine ortho-Phthlat freie Zusammensetzung angeboten werden kann.

## Patentansprüche

1. Gemisch von Bernsteinsäureestern,
**dadurch gekennzeichnet,**
**dass** der Alkylrest einen Anteil an Alkylkomponenten mit weniger als 9 C-Atomen von maximal 15 Massen-% aufweist,
der Alkylrest einen Anteil an Alkylkomponenten mit mehr als 9 C-Atomen von maximal 25 Massen-% aufweist,
wobei der Anteil an 3,5,5-Trimethylhexylresten bei maximal 5 mol-% und der Anteil an linearem n-Nonylresten bei maximal 15 mol-% liegt.

2. Gemisch nach Anspruch 1,
**dadurch gekennzeichnet,**
**dass** der Anteil der Bernsteinsäure bezogen auf alle Teile von Bernsteinsäure welche in dem Gemisch als Ester vorliegen, welcher auf nachwachsenden Rohstoffen basiert, mindestens 10 mol-% beträgt.

3. Gemische nach einem der Ansprüche 1 oder 2,
**dadurch gekennzeichnet,**
**dass** der Anteil der Bernsteinsäureester, die mindestens einen 3,5,5-Trimethylhexylrest enthalten, maximal 5 mol-% ist.

4. Gemische nach einem der Ansprüche 1 bis 3,
**dadurch gekennzeichnet,**
**dass** diese einen Anteil an Alkylresten mit 9 Kohlenstoffatomen, die eine Methylverzweigung am zweiten Kohlenstoffatom nach dem Sauerstoff der Carboxylgruppe aufweisen, von maximal 49,5 mol-% enthalten.

5. Gemische nach einem der Ansprüche 1 bis 4,
**dadurch gekennzeichnet,**
**dass** der Kochpunkt höher als 180° C liegt.

6. Gemische nach einem der Ansprüche 1 bis 5,
**dadurch gekennzeichnet,**
**dass** die Eigenviskosität bestimmt durch Scherrheometrie mit einer Scherrate von 1/s bei einer Temperatur von 20 °C maximal 40 mPa • s beträgt.

7. Verfahren zur Herstellung von Gemischen gemäß einem der Ansprüche 1 bis 6, umfassend das in Kontakt Bringen von Bernsteinsäure oder Bernsteinsäurederivaten mit einem isomerern Alkoholgemisch,
wobei hierdurch Wasser oder Methanol freigesetzt wird;
wobei ein bis zu 50% stöchiometrischer Überschuss des Alkoholgemisches eingesetzt wird; und
die Umsetzung unter Verwendung eines Katalysators erfolgt, ausgewählt aus der Gruppe umfassend Butyltitanat, Nonyltitanat.

8. Verfahren nach Anspruch 7,
wobei der Anteil der Bernsteinsäure oder Bernsteinsäurederivaten, welche zur Herstellung des Esters eingesetzt wird und auf nachwachsenden Rohstoffen basiert, mindestens 10 mol-% beträgt.

9. Zusammensetzung, enthaltend Gemische nach einem der Ansprüche 1 bis 6.

10. Zusammensetzung nach Anspruch 9,
wobei die Zusammensetzung zusätzlich mindestens einen Weichmacher enthält, ausgewählt aus der Gruppe der Alkylbenzoate, der Dialkyladipiate, der Glycerinester, der Citronensäuretrialkylester, der acylierten Citronensäuretrialkylester, der Trialkyltrimellitate, der Glykoldibenzoate, der Dialkylterephthalate, der Dialkylphthalate, den Dialkanoylestern des Isosorbid und/oder der Dialkylester der 1,2-, 1,3- oder 1,4-Cyclohexandicarbonsäuren.

11. Verwendung eines Gemisches nach einem der Ansprüche 1 bis 6 als Weichmacher.

12. Formteil enthaltend ein Gemisch nach einem der Ansprüchen 1 bis 6.

13. Fußbodenbelag enthaltend ein Gemisch nach einem der Ansprüchen 1 bis 6.

14. Wandbelag enthaltend ein Gemisch nach einem der Ansprüchen 1 bis 6.

15. Schutzmasse, Dichtmasse oder Klebstoff enthaltend ein Gemisch nach einem der Ansprüchen 1 bis 6.

16. Folie oder Plane enthaltend ein Gemisch nach einem der Ansprüchen 1 bis 6.

## Claims

1. Mixture of succinate esters,
**characterized in that**
the alkyl residue has a proportion of alkyl components with less than 9 carbon atoms of max. 15 wt%,
the alkyl residue has a proportion of alkyl components with more than 9 carbon atoms of max. 25 wt%,
wherein the proportion of 3,5,5-trimethylhexyl residues is max. 5 mol% and the proportion of linear n-nonyl residues is max. 15 mol%.

2. Mixture according to Claim 1,
**characterized in that**
the proportion of succinic acid relative to all parts of succinic acid that are present in the mixture as ester, which is based on renewable raw materials, is at least 10 mol%.

3. Mixtures according to one of Claims 1 or 2,
**characterized in that**
the proportion of succinate esters that contain at least one 3,5,5-trimethylhexyl residue is max. 5 mol%.

4. Mixtures according to one of Claims 1 to 3,
**characterized in that**
these contain a proportion of alkyl residues with 9 carbon atoms, which have a methyl branching on the second carbon atom after the oxygen of the carboxyl group, of max. 49.5 mol%.

5. Mixtures according to one of Claims 1 to 4,
**characterized in that**
the boiling point is above 180°C.

6. Mixtures according to one of Claims 1 to 5,
**characterized in that**
the intrinsic viscosity determined by shear rheometry with a shear rate of 1/s at a temperature of 20°C is max. 40 mPa • s.

7. Method of producing mixtures according to one of Claims 1 to 6, comprising contacting succinic acid or succinic acid derivatives with an isomeric alcohol mixture,
with liberation of water or methanol;
wherein an up to 50% stoichiometric excess of the alcohol mixture is used; and
the reaction takes place using a catalyst, selected from the group comprising butyl titanate, nonyl titanate.

8. Method according to Claim 7,
wherein the proportion of succinic acid or succinic acid derivative that is used for production of the esters and is based on renewable raw materials is at least 10 mol%.

9. Composition containing mixtures according to one of Claims 1 to 6.

10. Composition according to Claim 9,
wherein the composition additionally contains at least one plasticizer, selected from the group of alkyl benzoates, dialkyladipates, glycerol esters, trialkyl citrates, acylated trialkyl citrates, trialkyl trimellitates, glycol dibenzoates, dialkyl terephthalates, dialkyl phthalates, dialkanoyl esters of isosorbide and/or dialkylesters of 1,2-, 1,3- or 1,4-cyclohexane dicarboxylic acids.

11. Use of a mixture according to one of Claims 1 to 6 as plasticizer.

12. Molded article containing a mixture according to one of Claims 1 to 6.

13. Floor covering containing a mixture according to one of Claims 1 to 6.

14. Wall covering containing a mixture according to one of Claims 1 to 6.

15. Resist, sealant or adhesive containing a mixture according to one of Claims 1 to 6.

16. Film or canvas containing a mixture according to one of Claims 1 to 6.

## Revendications

1. Mélange d'esters de l'acide succinique, **caractérisé en ce que** le radical alkyle présente une proportion de composants alkyle présentant moins de 9 atomes de carbone d'au maximum 15% en masse, le radical alkyle présente une proportion de composants alkyle présentant plus de 9 atomes de carbone d'au maximum 25% en masse, la proportion de radicaux 3,5,5-triméthylhexyle étant d'au maximum 5% en mole et la proportion de radicaux n-nonyle linéaires étant d'au maximum 15% en mole.

2. Mélange selon la revendication 1, **caractérisé en ce que** la proportion d'acide succinique, par rapport à toutes les parties d'acide succinique qui se trouvent sous forme d'esters dans le mélange, qui est à base de matières premières renouvelables, est d'au moins 10% en mole.

3. Mélanges selon l'une quelconque des revendications 1 ou 2, **caractérisés en ce que** la proportion d'esters de l'acide succinique qui contiennent au moins un radical 3,5,5-triméthylhexyle est d'au maximum 5% en mole.

4. Mélanges selon l'une quelconque des revendications 1 à 3, **caractérisés en ce qu'**ils contiennent une proportion de radicaux alkyle comprenant 9 atomes de carbone, qui présentent une ramification méthyle sur le deuxième atome de carbone après l'oxygène du groupe carboxyle, d'au maximum 49,5% en mole.

5. Mélanges selon l'une quelconque des revendications 1 à 4, **caractérisés en ce que** le point d'ébullition est supérieur à 180°C.

6. Mélanges selon l'une quelconque des revendications 1 à 5, **caractérisés en ce que** la viscosité propre, déterminée par rhéométrie par cisaillement à une vitesse de cisaillement de 1/s à une température de 20°C est d'au maximum 40 mPa.s.

7. Procédé pour la préparation de mélanges selon l'une quelconque des revendications 1 à 6, comprenant la mise en contact d'acide succinique ou de dérivés de l'acide succinique avec un mélange d'alcools isomères, de l'eau ou du méthanol étant ainsi libéré(e) ; un excès stoechiométrique de jusqu'à 50% du mélange d'alcools étant utilisé ; et la transformation ayant lieu avec utilisation d'un catalyseur choisi dans le groupe comprenant le titanate de butyle, le titanate de nonyle.

8. Procédé selon la revendication 7, la proportion d'acide succinique ou de dérivés de l'acide succinique, qui est utilisée pour la préparation de l'ester et qui est à base de matières premières renouvelables, étant d'au moins 10% en mole.

9. Composition contenant des mélanges selon l'une quelconque des revendications 1 à 6.

10. Composition selon la revendication 9, la composition contenant en plus au moins un plastifiant, choisi dans le groupe des benzoates d'alkyle, des adipates de dialkyle, des esters de glycérol, des esters trialkyliques de l'acide citrique, des esters trialkyliques acylés de l'acide citrique, des trimellitates de trialkyle, des dibenzoates de glycol, des téréphtalates de dialkyle, des phtalates de dialkyle, des esters dialcanoyliques de l'isosorbide et/ou des esters dialkyliques de l'acide 1,2-cyclohexanedicarboxylique, 1,3-cyclohexanedicarboxylique ou 1,4-cyclohexanedicarboxylique.

11. Utilisation d'un mélange selon l'une quelconque des revendications 1 à 6 comme plastifiant.

12. Pièce façonnée contenant un mélange selon l'une quelconque des revendications 1 à 6.

13. Revêtement de sol contenant un mélange selon l'une quelconque des revendications 1 à 6.

14. Revêtement mural contenant un mélange selon l'une quelconque des revendications 1 à 6.

15. Masse de protection, masse d'étanchéité ou adhésif contenant un mélange selon l'une quelconque des revendications 1 à 6.

16. Feuille ou plan contenant un mélange selon l'une quelconque des revendications 1 à 6.
